# EUROPÄISCHE PATENTSCHRIFT

(11) **EP 3 273 898 B1**
(45) Veröffentlichungstag und Bekanntmachung des Hinweises auf die Patenterteilung: **24.02.2021**
(21) Anmeldenummer: 16712323.1
(22) Anmeldetag: 24.03.2016
(51) Int. Cl.: A61B 90/50, A61B 90/90, A61B 90/98, A61B 90/00, A61B 17/34, A61B 34/20, A61B 17/29

(54) **CHIRURGISCHES INSTRUMENT SOWIE SYSTEM**
SURGICAL INSTRUMENT AND SYSTEM
INSTRUMENT CHIRURGICAL ET SYSTÈME

(30) Priorität: 27.03.2015 DE 102015104821
(43) Veröffentlichungstag der Anmeldung: 31.01.2018
(73) Patentinhaber: medineering GmbH, 81829 München (DE)
(72) Erfinder: NOWATSCHIN, Stephan, 81677 München (DE); KRINNINGER, Maximilian, 82234 Weßling-Oberpfaffenhofen (DE); GIERLACH, Dominikus, 80798 München (DE)
(74) Vertreter: Eisenführ Speiser
(86) Internationale Anmeldenummer: PCT/EP2016/056556
(87) Internationale Veröffentlichungsnummer: WO 2016/156201

(56) Entgegenhaltungen:
- EP-A1- 1 517 119
- EP-A1- 1 517 119
- EP-A1- 1 517 119
- EP-A1- 1 958 587
- EP-A1- 1 958 587
- EP-A1- 2 921 110
- EP-A1- 2 921 110
- EP-A2- 1 520 548
- EP-A2- 1 520 548
- WO-A1-2015/012082
- WO-A1-2015/012082
- DE-A1- 19 750 698
- DE-A1- 19 750 698
- DE-A1-102008 033 740
- DE-A1-102008 033 740
- DE-A1-102013 100 605
- DE-A1-102013 100 605
- US-A1- 2005 107 669
- US-A1- 2005 107 669
- US-A1- 2012 190 987
- US-A1- 2012 190 987
- US-A1- 2012 190 987
- US-A1- 2013 110 288
- US-A1- 2013 110 288
- US-A1- 2013 204 095
- US-A1- 2013 204 095
- US-A1- 2013 325 030
- US-A1- 2013 325 030
- US-A1- 2014 163 736
- US-A1- 2014 163 736
- US-A1- 2014 213 892
- US-A1- 2014 213 892

## Beschreibung

Die Erfindung betrifft ein chirurgisches Instrument, insbesondere endoskopisches Instrument, mit einem Schaft zur Einführung in eine Körperöffnung eines Patienten, wobei der Schaft der einen distalen und einen proximalen Abschnitt aufweist, wobei am distalen Abschnitt eine Eingriffseinheit angeordnet ist, sowie einen chirurgischen Manipulator zum Halten und Führen des Instruments, der vorzugsweise an einem Haltearm befestigbar und mit diesem positionierbar ist, ein chirurgisches System mit wenigstens zwei Manipulatoren und zwei Instrumenten, wobei jeweils ein Instrument in einem Manipulator aufgenommen ist.

In der modernen Chirurgie werden vermehrt minimal invasive Instrumente verwendet, insbesondere endoskopische Instrumente, die in natürliche oder künstliche Öffnungen des Patientenkörpers eingeführt werden. Diese sind von außerhalb des Patientenkörpers nur mit speziellen Geräten sichtbar, nicht jedoch mit dem bloßen Auge. Bei der Verwendung von robotischen Assistenzsystemen ist es erforderlich, die Lage des robotischen Assistenzsystems sowie von diesem getragenen Assistenzsystemen und/oder Instrumenten zu kennen, als auch die von einem Operateur manuell geführten Instrumente. Dies kann durch eine globale dreidimensionale Bilderfassung und Verarbeitung erfolgen, was allerdings in der Regel bedingt, den Patienten einer erheblichen elektromagentischen Strahlung auszusetzen.

Bisher existieren keine befriedigenden Lösungen die manuell vom Chirurgen geführten und/oder ins Operationsfeld eingebrachten Instrumente zu erfassen und deren Lage in Bezug auf den Patienten und/oder ein Referenzsystem, wie etwa ein chirurgisches Navigationssystem, zu bestimmen. Zwar existieren beispielsweise Reflektoren, die von OP-Kameras aufgenommen werden, und die an den Instrumenten angebracht sind. Mittels dieser Reflektoren ist dann die Position eines Instruments im OP-Saal innerhalb des Navigationsfelds bestimmbar. Allerdings kann auch mit solchen Reflektoren eine Position des Instruments innerhalb des Patientenkörpers nur schwer bestimmt werden.

Das Dokument EP 1 517 119 A1 offenbart eine Vorrichtung zum Bestimmen einer longitudinalen Position und einer Winkelposition eines rotationssymmetrischen Schafts. Zur Bestimmung der Winkelposition ist eine schraubenförmige Markierung vorgesehen, die optisch erfasst wird.

Das Dokument US 2012 0190 987 A1 offenbart eine Vorrichtung zum Bestimmen einer Bewegungsdistanz einer Nadel. Eine Winkelposition wird jedoch nicht erfasst.

Das Dokument WO 2015 012082 A1 offenbart ein System aufweisend ein medizinisches Instrument mit einer Antriebseinrichtung zur Einstellung der Winkelposition eines Endeffektors bezüglich eines Schafts, einen Trokar zum Einführen des medizinischen Instruments, einen Sensor zur Erfassung der Winkelposition sowie eine Steuerungseinheit zum Ansteuern der Antriebseinrichtung auf der Grundlage von Signalen des Sensors.

Das Dokument DE 10 2008 033 740 A1 offenbart ein chirurgisches Instrument mit einer Einführtiefendetektionseinrichtung zum taktilen Erkennen einer Einführtiefe des Instruments in einen Führungskanal.

Die Aufgabe der vorliegenden Erfindung ist es, ein Instrument, ein Manipulator sowie ein chirurgisches System anzugeben, mit denen die erläuterten Probleme wenigstens teilweise behoben sind.

Die Aufgabe wird gelöst durch die Gegenstände der unabhängigen Ansprüche 1 und 3.

In einem ersten Aspekt der Erfindung wird die eingangs genannte Aufgabe bei einem Instrument in der eingangs genannten Art gelöst durch die Merkmale des Anspruchs 1, wobei der Schaft des Instruments eine Maßverkörperung aufweist, die mittels einer Sensoreinrichtung zum Bestimmen einer Eindringtiefe und/oder Drehposition des Instruments abtastbar ist.

Instrumente, die zur minimalen invasiven Chirurgie oder endoskopischen Chirurgie verwendet werden weisen einen länglichen Schaft auf, der starr oder flexibel ist. Am distalen Ende ist eine Eingriffseinheit angeordnet, die beispielsweise eine Klemme oder eine Einrichtung zur Biopsie, als auch eine Optik von einem Endoskop sein kann. Die Eingriffseinheit wird durch eine natürliche oder künstliche Körperöffnung bis an einen gewünschten Punkt innerhalb des Patientenkörpers geführt, um dort die erforderliche Operation und Aufgabe auszuführen. Indem der Schaft eine Maßverkörperung aufweist, die mittels einer Sensoreinrichtung zum Bestimmen einer Eindringtiefe und/oder Drehposition des Instruments abtastbar ist, ist die Position des chirurgischen Instruments relativ zu der Sensoreinrichtung erfassbar und, wenn die Position des Sensorsystems innerhalb eines chirurgischen Navigationssystems oder in Bezug auf ein Referenzsystem wie beispielsweise den Patienten bekannt ist, ist auch die Lage des chirurgischen Instruments in Bezug auf dieses Referenzsystem und/oder dem Patienten bekannt. Beispielsweise ist die Sensoreinrichtung benachbart zu der natürlichen oder künstlichen Körperöffnung, durch die das Instrument eingeführt wird angeordnet, wie weiter unten genauer erläutert werden wird.

Die Maßverkörperung weist eine Mehrzahl an um eine Zentralachse des Schafts angeordnete Breitengrade auf. Die Breitengrade sind vorzugsweise als Ringe ausgebildet, deren Zentralachse bevorzugt koaxial zu einer Zentralachse des Instruments angeordnet ist. Alternativ sind die Ringe leicht schräg, sodass die Zentralachse der Ringe einen Winkel zur Zentralachse des Instruments aufweist. Vorzugsweise ist der Abstand der einzelnen Breitengrade so gewählt, dass eine ausreichende Auflösung erreicht wird. Bevorzugt liegt die Auflösung im Millimeterbereich, d.h. die einzelnen Breiten- und/oder Längengrade habe einen Abstand von 1 mm oder weniger, vorzugsweise 0,8mm oder weniger, weiter vorzugsweise 0,5mm oder weniger, besonders bevorzugt 0,1 mm oder weniger. In der Regel reicht eine Auflösung von 1 mm für die meisten operativen Anwendungen aus. Eine derartige Auflösung ist auch kostengünstig herstellbar. Die einzelnen Breiten- und/oder Längengrade sind vorzugsweise identisch ausgebildet und bevorzugt äquidistant zueinander. Mittels solcher Breitengrade ist insbesondere eine Eindringtiefe des Instruments erfassbar. Es kann die axiale Position relativ zu einem Sensor, beispielsweise ringförmigen Sensor, durch eine ringförmige Sensoreinrichtung, durch die das Instrument verläuft bzw. hindurch geschoben wird, erfasst werden.

In einer Alternative ist wenigstens eine erste Mehrzahl der Breiten- und/oder Längengrade nicht äquidistant zueinander angeordnet, sondern mit vorbestimmten, unterschiedlichen Abständen und/oder Stärke ausgebildet. Bevorzugt ist diese erste Mehrzahl am proximalen Ende angeordnet. Die erste Mehrzahl umfasst vorzugsweise etwa 5 oder 10 Breiten- und/oder Längengrade. Hierdurch ist es möglich eine Kodierung des Instruments vorzusehen, welche bei einem Anordnen des Instruments, beispielsweise in einem Trokar, und Vorbeiführen an einer entsprechenden Sensoreinrichtung abtastbar ist. Die Sensoreinrichtung kann dazu mit einer Erkennungseinheit zum Erkennen der Identität des Instruments gekoppelt sein. Die erkannte Identität kann dann in ein OP-Navigationssystem zurückgespielt werden und/oder für die (teil-)automatisierte Erstellung eines OP-Protokolls verwendet werden.

Die Maßverkörperung weist zusätzlich (oder alternativ) eine Mehrzahl an parallel zu einer Zentralachse des Schafts angeordneten Längengraden auf. Die Längengrade können ebenfalls leicht schräg zu einer Zentralachse des Schafts ausgebildet sein. Sind sowohl die Breitengrade als auch die Längengrade schräg zu einer Längsachse ausgerichtet, jedoch senkrecht zueinander, ist mit einer entsprechenden Auswertung eine genaue Erfassung möglich. Die Längengrade dienen insbesondere dazu, eine Drehposition bezogen auf die Zentralachse des Instruments mit der Sensoreinrichtung zu erfassen. Wenn die Maßverkörperung sowohl Längengrade als auch Breitengrade aufweist, ist sowohl eine Eindringtiefe als eine Drehposition des Instruments auf einfache Art und Weise erfassbar.

In einer bevorzugten Weiterbildung ist die Maßverkörperung optisch abtastbar. Die Maßverkörperung ist gemäß diesem Ausführungsbeispiel vorzugsweise auf einer äußeren Oberfläche des Schafts des Instruments aufgebracht. Beispielweise ist die Maßverkörperung in Form von Linien, insbesondere farblich abgesetzte Linien aufgebracht. Alternativ oder zusätzlich ist die Maßverkörperung in Form von Erhebungen oder Rillen auf der Oberfläche des Schafts aufgebracht. Dies ist eine besonders einfache Möglichkeit der dauerhaften Aufbringung der Maßverkörperung, welches zudem gut durch eine optische Sensoreinrichtung erfassbar ist.

Die Maßverkörperung ist elektromagnetisch abtastbar. Die Maßverkörperung, aufweisend insbesondere Längen und Breitengrade, ist in Form von magnetisierten Abschnitte auf dem Schaft aufgebracht. Das heißt, die Oberfläche des Schafts, der gemäß dieser Ausführungsform vorzugsweise auf einem Metall gebildet ist, weist ringförmig magnetisierte Abschnitte und magnetisierte Längsstreifen auf, die Breiten- und Längengrade der Maßverkörperung bilden. Eine solche Maßverkörperung ist besonders bevorzugt, da die Oberfläche insgesamt glatt ausgebildet sein kann und daher für Verschmutzung unanfällig ist. Ferner ist eine solche magnetisierte Maßverkörperung weitgehend unabhängig von einer Verschmutzung des Instrumentenschaft, beispielsweise mit Körperflüssigkeiten, abtastbar, was bevorzugt ist.

Gemäß einem zweiten Aspekt ist ein Adapter für ein chirurgisches Instrument vorgesehen, wobei der Adapter dazu angepasst ist auf einen einem Schaft des chirurgischen Instruments zur Einführung in eine Körperöffnung eines Patienten, der einen distalen und einen proximalen Abschnitt aufweist, wobei am distalen Abschnitt eine Eingriffseinheit angeordnet ist, befestigt zu werden, wobei der Adapter eine Maßverkörperung aufweist, die mittels einer Sensoreinrichtung zum Bestimmen einer Eindringtiefe und/oder Drehposition des Instruments abtastbar ist. Der Adapter weist vorzugsweise einen in etwa hülsenförmigen Grundkörper auf, der auf das Instrument aufschiebbar ist, oder ist als eine Folie ausgebildet, die auf den Schaft des Instruments aufbringbar ist. Der Adapter wird bevorzugt an einer vorbestimmten, bekannten Position an dem Instrument angeordnet. Hierzu können entsprechende Positionierungsmittel, etwa eine Rastverbindung mit Rastnase und Rastnut zur definierten Positionierung des Adapters an dem chirurgischen Instrument vorgesehen sein. Es soll verstanden werden, dass der Adapter gemäß dem zweiten Aspekt und das chirurgische Instrument gemäß dem ersten Aspekt der Erfindung gleiche und ähnliche Weiterbildungen haben, wie sie insbesondere in den abhängigen Ansprüchen zum chirurgischen Instrument gemäß dem ersten Aspekt der Erfindung niedergelegt sind. Insofern wird vollumfänglich auf die obige Beschreibung Bezug genommen.

In dem dritten Aspekt der Erfindung wird die eingangs genannte Aufgabe bei einem chirurgischen Manipulator zum Halten vom Führen eines Instruments nach einer der vorstehend beschriebenen bevorzugten Ausführungsformen eines chirurgischen Instruments gelöst durch eine Sensoreinrichtung, mittels der die Maßverkörperung auf dem Schaft des Instruments zum Bestimmen einer Eindringtiefe und/oder Drehposition des Instruments abtastbar ist, gemäß der Merkmale von Anspruch 3. Der chirurgische Manipulator ist vorzugsweise an einem Halterarm befestigbar. Der Halterarm kann eine passiver oder ein aktiver Halterarm sein, der den Manipulator an beispielsweise einem Operationstisch abstützt. Der Manipulator selbst kann wiederum aktiv oder passiv sein und insbesondere wie in EP 1 658 016 B1 offenbart ausgebildet sein, oder wie in der Dissertation von Dr. Maximilian Krinninger mit dem Titel "Ein System zur Endoskopführung in der HNO-Chirurgie", Lehrstuhl für Mikrotechnik und Medizingerätetechnik, technische Universität München, 2010 dargestellt ausgebildet sein. Der Manipulator ist demnach ausgebildet, das Instrument relativ zum Halterarm zu verschwenken.

Bevorzugt weist die Sensoreinrichtung des chirurgischen Manipulators wenigstens eine Kamera zum optischen Erfassen der Maßverkörperung auf. Eine solche Kamera kann beispielsweise eine Lichtquelle mit entsprechendem Sensor, einen Laserscanner oder dergleichen aufweisen oder als solche ausgebildet sein, um die Maßverkörperung optisch abzutasten. Auch ein oder mehrere CCD Elemente sind hierbei bevorzugt.

In einer Alternative oder zusätzlich weist die Sensoreinrichtung wenigstens ein Magnet-Sensorkopf zum magnetosensitiven Erfassen der Maßverkörperung auf. Ist die Maßverkörperung wie oben dargelegt mittels regelmäßig magnetisierter Abschnitte auf den Instrumentenschaft ausgebildet, ist ein solcher Sensor bevorzugt. Derartige Sensoren sind beispielsweise von der Fa. Baumer Elektrik AG, Schweiz, unter den Namen "HDmag" erhältlich. Dies ist eine besonders bevorzugte Ausführungsform, da in Kombination mit der oben beschriebenen elektromagnetisch abtastbaren Maßverkörperung die Abtastung der Maßverkörperung durch den Magnet-Sensorkopf unabhängig von etwaigen Fluiden oder Verschmutzungen auf der Oberfläche des Instrumentenschafts ist.

In einer weiteren bevorzugten Ausführungsform weist der Manipulator einen Trokar zur Aufnahme des Schafts des Instruments auf, wobei die Sensoreinrichtung an dem Trokar, insbesondere einer proximalen Öffnung des Trokars angeordnet ist. Der Trokar dient zur Führung des Instrumentenschafts und zu seiner Halterung. An dem Trokar ist die Sensoreinrichtung angeordnet. Vorzugsweise weist die Sensoreinrichtung eine in etwa ringförmige Struktur auf, die an einem proximalen Ende des Trokars angeordnet ist. Die Lage des Trokars in einem OP-Navigationssystem ist bekannt, entweder aufgrund des Halterarms, dessen Pose bekannt ist, oder aufgrund von an dem Trokar angeordneten Reflektoren für ein OP-Navigationssystem. Da die Lage des Trokars bekannt ist, ist mit dieser Ausführungsform auch die Lage des Instruments, sowohl axial als auch sein Drehwinkel bekannt. Auf diese Weise lässt sich auf einfach Art und Weise auch die Eindringtiefe des Instruments bestimmen, wenn der Trokar benachbart zu einer natürlichen oder künstlichen Öffnung des Patientenkörpers oder auch in dem Patientenkörper angeordnet ist. Ferner ist so auch die Position des Instruments, auch wenn dieses manuell innerhalb des Trokars axial und rotatorisch bewegt wird, bekannt, sodass die Lage des Instruments zu weiteren im Operationsfeld befindlichen Elementen auf einfache Art und Weise bestimmt werden kann. Hierdurch ist die Sicherheit während der Operation wesentlich verbessert. Ferner kann auf diese Weise auch eine Dokumentation des Operationsablaufs erfolgen, bspw. durch Protokollierung der Position der Instrumente.

Gemäß einer weiteren bevorzugten Ausführungsform ist die Sensoreinrichtung nahe an einer Körperöffnung, insbesondere natürlichen Körperöffnungen des Patienten positioniert. Die Sensoreinrichtung kann dazu an einem Adapter angeordnet sein, der an oder um die Körperöffnung herum an den Patienten befestigbar ist. Ein solcher Adapter kann beispielsweise grundsätzlich wie das Produkt ViKY oder LER der Fa. Endocontrol-Medical, La Tronche, Frankreich, oder auch das sogenannte P-Arm System von Taniguchi oder Sekimoto ausgebildet sein. Diese Systeme sind chirurgische Manipulatoren, die unmittelbar auf dem Patientenkörper aufgebracht werden und ein Instrument und oder einen Trokar halten. Mittels der Sensoreinrichtung ist nun die Eindringtiefe und Rotation des Instruments bezogen auf den Patientenkörper auf einfach Weise erfassbar.

Gemäß einer weiteren bevorzugten Ausführungsform weist der Manipulator eine Antriebs- und/oder Bremseinrichtung zum Antreiben und/oder Bremsen einer Bewegung des Instruments relativ zum Manipulator auf. Eine Bremseinrichtung ist besonders bevorzugt, wenn das Instrument manuell bezogen auf den Manipulator bewegt wird. Die Bremseinrichtung kann beispielsweise mit einer Auswerteeinheit und einer Steuereinheit gekoppelt sein. Erkennt die Auswerteeinheit, aufgrund der Eindringtiefe und der Drehposition des Instruments, dass ein Konflikt mit weiteren Instrumenten oder eine Verletzung des Patienten droht, kann die Steuereinheit die Bremseinrichtung veranlassen, eine Bewegung des Instruments relativ zum Manipulator zu bremsen und oder zu stoppen. Dies wird möglich, da aufgrund der Maßverkörperung auf den Instrument und der Sensoreinrichtung am Manipulator eine Lage des Instruments permanent bekannt und erfassbar ist. Hierdurch ist die Sicherheit wesentlich verbessert.

Eine Antriebseinrichtung ist besonders dann bevorzugt, wenn der Manipulator an einem (passiven) Halterarm angeordnet ist. Bewegt ein Operateur manuell ein weiteres, zweites Instrument, in Richtung des an dem Manipulator an dem Halterarm aufgenommenen Instruments, kann die Antriebseinheit durch eine Steuereinheit veranlasst werden das Instrument anzutreiben und es beispielsweise ein Stückchen aus dem Patientenkörper herauszufahren. Ein solcher Anwendungsfall ist beispielsweise gegeben, wenn ein Operateur manuell eine Fräse in ein Operationsfeld einbringt, und als Instrument an dem Manipulator an dem Halterarm ein Endoskop zur Beobachtung des Operationsfelds angeordnet ist. Wird nun durch den Operateur beim Ausführen einer Fräsoperation zusätzlich ein Sauger eingebracht, kann es bevorzugt sein, das Endoskop um einen kleinen Weg, beispielsweise 5 Millimeter, von Operationsfeld wegzufahren, um auch beim Fräsen und Absaugen eine optimale Sicht zu gewährleisten. Aufgrund der Maßverkörperung und der Sensoreinrichtung am Manipulator ist die Position des Endoskops bekannt, und auch die Position des Endoskops relativ zu der Fräse und dem Sauger, sodass die Antriebseinrichtung entsprechend gesteuert werden kann.

Die Sensoreinrichtung ist mit einer Steuereinheit verbunden, die dazu eingerichtet ist, eine Funktion des Manipulators und/oder des Instruments in Abhängigkeit von durch die Sensoreinrichtung erfassten Werten zu sperren oder freizugeben. Ist aufgrund der Maßverkörperung und der Sensoreinrichtung die Lage des Instruments im Patientenkörper bekannt, kann eine solche Sperrung oder Freigabe bevorzugt sein. Ist beispielsweise als Instrument eine Schere vorgesehen, kann eine Öffnen- und Schließbewegung der Schwere solange gesperrt werden, wie sich die Schere nicht an einem vorgesehenen gewünschten Ort befindet, der mittels der Maßverkörperung der entsprechenden Sensoreinrichtung erfasst und verifiziert wird. Hierdurch wird eine Fehlbedienung und ein versehentliches Öffnen und Schließen der Schere verhindert. Gleiches gilt für andere Instrumente, wie Sauger, Fräser oder Nähwerkzeuge.

Gemäß einem vierten Aspekt der Erfindung wird die eingangs genannte Aufgabe bei einem chirurgischen System mit wenigstens zwei Manipulatoren gemäß einer der bevorzugten Ausführungsformen eines Manipulators gemäß dem dritten Aspekt der Erfindung sowie zwei Instrumenten gemäß einer der vorstehend beschriebenen bevorzugten Ausführungsformen eines Instruments gemäß dem ersten Aspekt der Erfindung, wobei jeweils ein Instrument in einem Manipulator aufgenommen ist, dadurch gelöst, dass die Sensoreinrichtungen der Manipulatoren mit einer Steuereinheit verbunden sind, wobei die Steuereinheit dazu ausgebildet ist, eine Funktion des ersten Manipulators und/oder des ersten Instruments in Abhängigkeit von durch die ersten und zweiten Sensoreinrichtungen erfassten Werte des ersten und zweiten Instruments zu sperren oder freizugeben. Beispielsweise kann vorgesehen sein, dass wenn eine Kollision der beiden Instrumente droht, wenigstens einer der Manipulatoren den weiteren Vorschub wenigstens eines Instruments bremst oder sperrt, sodass die Kollision verhindert wird. Ein weiteres Beispiel ist, wenn ein Instrument eine Klemme ist und das andere Instrument eine Schere. Die Klemme ist vorgesehen, einen speziellen Gewebeabschnitt des Patienten zu klemmen, während die Schere diesen Gewebeabschnitt schneiden soll. Es kann vorgesehen sein, dass die Schere erst öffnen- und schließbar ist, wenn sich die Klemme an einer speziellen vorbestimmten Position befindet und geschlossen ist. Hierdurch ist die Sicherheit erhöht.

Gemäß einem fünften Aspekt, der nicht Bestandteil der beanspruchten Erfindung ist, ist ein Haltearm zum Aufnehmen eines Manipulators gemäß dem dritten Aspekt der Erfindung ausgebildet, mit wenigstens einem ersten und einem zweiten Armsegment, wobei das erste Armsegment mit einem ersten Gelenk und das zweite Armsegment mit einem zweiten Gelenk verbunden ist, wobei jedes Gelenk freigebbar und arretierbar ist. Vorzugsweise ist der Manipulator an dem Haltearm angeordnet.

Der Haltearm selbst ist dabei bevorzugt wie in DE 10 2014 016 824 oder DE 10 2014 016 823 der hiesigen Anmelderin ausgebildet. Insofern wird vollumfänglich auf diese Offenbarungen verwiesen. Ein solcher Haltearm weist demnach vorzugsweise eine Bedieneinrichtung zum Verbringen des Haltearms in eine gewünschte Pose auf, wobei die Bedieneinrichtung dazu eingerichtet ist, bei Kontakt zwischen einer Bedieneinrichtung dazu eingerichtet, bei Kontakt zwischen einer Bedienperson und einem der ersten und zweiten Armsegmente das zugeordnete Gelenk freizugeben. Demnach ist bevorzugt vorgesehen, dass die Bedieneinrichtung dazu eingerichtet ist, bei Kontakt zwischen einer Bedienperson und dem ersten Armsegment das erste Gelenk freizugeben und bei Kontakt zwischen einer Bedienperson und dem zweiten Armsegment das zweite Gelenk freizugeben. Es ist daher vorgesehen, dass bei Kontakt einer Bedienperson mit einem entsprechenden nur das zugeordnete Gelenk freigegeben wird. Dadurch ist es möglich, intuitiv, einzelne Gelenke zu bewegen und den Haltearm so segmentweise zu verstellen und in eine gewünschte Pose zu bringen. Dadurch ist eine genau Positionierung des Haltearms und dadurch auch des Manipulators und des Instruments möglich, da inkrementell jedes Segment separat verstellt werden kann. Es ist ebenso möglich, mehrere Segmente auf einmal zu kontaktieren, sodass mehrere Gelenke gleichzeitig freigegeben werden und so verstellbar sind. Dadurch ist es möglich, den Haltearm auf einfache Art und Weise, insbesondere intuitiv, in eine gewünschte Pose zu überführen.

Neben den ersten und zweiten Armsegmenten sind vorzugsweise weitere Armsegmente vorgesehen, die ebenfalls jeweils einem Gelenk zugeordnet sind. Die Armsegmente selbst sind im Wesentlichen starr und vorzugsweise im Wesentlichen stabförmig. Der Begriff "stabförmig" umfasst hier sowohl im Wesentlichen gerade Armsegmente als auch leicht bis stark gekrümmte Armsegmente. Bei einem derartigen Haltearm wechseln sich Armsegmente und Gelenke stets ab, wobei der Haltearm am distalen und am proximalen Ende sowohl mit einem Gelenk als auch mit einem Segment oder einem Anschlusselement enden kann. Mit dem proximalen Ende ist der Haltearm an einer Basis befestigbar. Die Basis kann alternativ fest mit dem Arm gekoppelt sein, oder der Arm ist von der Basis entnehmbar. Die Basis ist in einer Ausführungsform als Operationstisch ausgebildet, und der Haltearm ist mit einem Operationstisch koppelbar. Vorzugsweise ist der Haltearm mit einer an dem Operationstisch vorgesehenen Normschiene koppelbar. Derartige Normschienen sind in der Regel bei Operationstischen vorgesehen, sodass an dem Haltearm zur Kopplung mit der Normschiene eines Operationstisches eine Standardschnittstelle vorgesehen sein kann. Übliche Operationstische sind zudem aus einzelnen Segmenten zusammengesetzt. Zur Kopplung weisen sie Segmente an den Stirnseiten entsprechende Koppelstellen auf, die in der Regel herstellerspezifisch sind. Bevorzugt ist der Haltearm über eine solche Koppelstelle an dem Operationstisch befestigbar. Dazu kann an dem proximalen Ende ein herstellerspezifischer Adapter vorgesehen sein. Alternativ ist die Basis als eine separate Vorrichtung, wie etwa ein Ständer, der auf dem Boden eines Operationssaales aufstellbar ist. In einer weiteren Alternative ist die Basis als eine Halterung ausgebildet, die beispielsweise an einer Wand oder Decke eines Operationssaales befestigbar ist.

Der Haltearm ist vorzugsweise als sogenannter passiver Haltearm ausgebildet und weist daher ausschließlich aktiv gebremste Gelenke auf, jedoch keine angetriebenen Gelenke, wie dies bei robotischen Haltearmen häufig der Fall ist. Jedes Gelenk ist daher nur freigeb- und arretierbar, allerdings nicht antreibbar. Hierdurch ist der Haltearm einfach aufgebaut und bedarf keiner komplexen Steuerung zu seinem Betrieb.

Gemäß einer bevorzugten Ausführungsform des Haltearms weist die Bedieneinrichtung Kontaktmittel auf, die dazu vorgesehen sind, dass ein Bediener mit diesen in Kontakt kommt, wobei ein erstes Kontaktmittel der Bedieneinrichtung an dem ersten Armsegment angeordnet ist und ein zweites Kontaktmittel an dem zweiten Armsegment angeordnet ist. Bei Kontakt mit dem ersten Kontaktmittel wird vorzugsweise das erste Gelenk freigegeben, und bei Kontakt mit dem zweiten Kontaktmittel wird vorzugsweise das zweite Gelenk freigegeben. Die Kontaktmittel dienen dazu, den Kontakt zwischen dem Benutzer und dem Armsegment zu erfassen. Die Kontaktmittel sind vorzugsweise jeweils an einer Oberfläche des entsprechenden Armsegments angeordnet. Die Kontaktmittel können sich über das gesamte Armsegment erstrecken oder nur einen Abschnitt von diesem einnehmen. Vorzugsweise erstreckt sich das Kontaktmittel jeweils in etwa um einen halben Umfang bezogen auf eine Zentralachse eines Armsegments. Dadurch ist das Kontaktmittel in jeder Pose des Haltearms leicht zugänglich, und ein Bediener kann leicht mit diesem in Kontakt kommen.

Weiterhin weist der Haltearm vorzugsweise am distalen und proximalen Ende Schnittstellen auf, die zur Kopplung eines Manipulators am distalen Ende und zur Übergabe von Daten und Energie an und von dem Manipulator ausgebildet ist, sowie eine Schnittstelle am proximalen Ende des Haltearms, über den der Haltearm mit Daten und Energie versorgbar ist und vorzugsweise an ein herkömmliches OP-Navigationssystem anschließbar ist. Dazu weist der Haltearm im Inneren vorzugsweise ein (Daten-)Bussystem auf, das die beiden Schnittstellen und einzelne Gelenke miteinander koppelt, und über das auch die Sensoreinrichtung an dem Manipulator mit Energie versorgt wird und mit einer entsprechenden Auswerteeinheit sowie einer Steuereinheit gekoppelt ist.

Gemäß einem sechsten Aspekt, welcher nicht Bestandteil der beanspruchten Erfindung ist, wird ein Verfahren zum Steuern eines chirurgischen Systems offenbart, insbesondere einem vorstehend beschriebenen bevorzugten System gemäß dem vierten Aspekt der Erfindung, wobei das Verfahren folgende Schritte aufweist: Bestimmen einer ersten Position eines ersten Instruments zu einem Referenzsystem; Bestimmen einer ersten Position eines zweiten Instruments zu dem Referenzsystem; Erfassen einer gewünschten zweiten Position, in die das erste Instrument zu bewegen ist; und Bestimmen, ob in der zweiten Position des ersten Instruments ein Konflikt mit dem zweiten Instrument vorliegt. Bevorzugt weist das Verfahren ferner den Schritt auf: Bestimmen einer erforderlichen zweiten Position und/oder Bewegung des zweiten Instruments, zum Verhindern eines Konflikts der ersten und zweiten Instrumente, wenn das erste Instrument in die zweite Position des ersten Instruments bewegt wird. Ein solcher Konflikt kann beispielsweise die Kollision der beiden Instrumente sein. Wird erkannt, dass das erste Instrument von der ersten Position in die zweite Position bewegt wird und wird gleichzeitig erkannt, dass dann eine Kollision der beiden Instrumente auftreten würde, wird gemäß diesem Verfahren für das zweite Instrument eine zweite Position und/oder Bewegung bestimmt, die eingenommen beziehungsweise ausgeführt werden muss, um den Konflikt zu verhindern. Es kann vorgesehen sein, dass das Verfahren ferner den Schritt aufweist, Bewegen des zweiten Instruments in die zweite Position. Dies kann manuell erfolgen, oder durch eine entsprechende Antriebseinheit in einem entsprechenden Manipulator.

Vorzugsweise weist das Verfahren den Schritt auf: Sperren oder Freigeben wenigstens einer Funktion des ersten und/oder zweiten Instruments. Dies erfolgt bevorzugt auf Basis des Bestimmung, ob in der zweiten Position des ersten Instruments ein Konflikt mit dem zweiten Instrument vorliegt.

Ein Beispiel für die Anwendung ist das Arbeiten mit Koagulationsinstrumenten. Ist eines der beiden ersten und zweiten Instrumente ein Koagulationsinstrument, beispielsweise eine Klemme zum Veröden, und das andere beispielsweise ein Endoskop, ist es wünschenswert, wenn sich diese Instrumente nicht berühren oder nicht sehr nah beieinander sind, da sonst ein eventueller Strom oder Lichtbogen von der Klemme auf das Endoskop überspringen kann. Liegt ein solcher Konflikt vor, wird dies gemäß dem Verfahren bestimmt. Auf Basis dieser Bestimmung wird bevorzugt ein Signal erzeugt, welches angibt, dass ein Konflikt besteht, oder bestehen wird und dieses Signal wird einer Steuereinheit des ersten und/oder zweiten Instruments bereitgestellt. Die Steuereinheit sperrt auf Basis dieses Signal dann eine oder mehrere Funktionen des entsprechenden Instruments, oder gibt sie frei. In dem Beispiel wird demgemäß der Stromfluss für die Koagulation gesperrt. Dieser wird erst wieder freigegeben, wenn der Konflikt nicht mehr besteht.

Ein weiteres Beispiel ist das Nähen mit zwei laparoskopischen Instrumenten. Hier besteht ein Konflikt, wenn die beiden Instrumente nicht nah genug beieinander sind. Auf Basis des bestimmten Konflikts, wird in einem solchen Fall vorzugsweise die Funktion der Übergabe einer Nadel an einen Nadelhalter gesperrt. Die Funktion wird erst freigegeben, wenn die Übergabe rechnerisch möglich ist. Das heißt, die Greifbranchen des nadelführenden Instruments lassen sich erst offen, wenn ein zweites, übernehmendes Instrument in der Nähe ist.

Im Falle einer chirurgischen Fräse (erstes Instrument) kann die Fräse abgeschaltet werden, sobald ein Konflikt mit einem zweiten Instrument bestimmt wird. Dadurch wird das zweite Instrument (z.B. Endoskop-Optik) nicht beschädigt. Umgekehrt kann im Falle des Einführens des zweiten Instruments die Bewegungsrichtung visualisiert werden, um das zweite Instrument möglichst schnell in das Operationsgebiet zu bewegen, wo sich bereits das erste Instrument befindet. Dies ist zum Beispiel der Fall, wenn das zweite Instrument gewechselt wurde und ein neues Instrument eingeführt werden muss. Dann kann es, je nach Erfahrung des Chirurgen, lange dauern, bis das neue Instrument am OP-Gebiet ist.

Es soll verstanden werden, dass das chirurgische Instrument gemäß dem ersten Aspekt, der Adapter gemäß dem zweiten Aspekt, der chirurgische Manipulator gemäß dem dritten Aspekt und das chirurgische System gemäß dem vierten Aspekt eine Mehrzahl an gleichen und ähnlichen bevorzugten Ausführungsformen aufweisen, wie sie insbesondere in den Unteransprüchen niedergelegt sind.

Nachfolgend wird die Erfindung anhand von Ausführungsbeispielen unter Bezugnahme auf die beiliegenden Figuren näher erläutert werden. Dabei zeigen:
- Figur 1: ein chirurgisches Instrument gemäß einem ersten Ausführungsbeispiel;
- Figur 2: ein chirurgisches Instrument gemäß einem zweiten Ausführungsbeispiel;
- Figur 3: einen Manipulator gemäß einem ersten Ausführungsbeispiel,
- Figur 4: eine Seitenansicht eines Haltearms;
- Figur 5: eine teilweise aufgebrochene Ansicht des Haltearms aus Figur 4;
- Figur 6: eine schematische Darstellung des vierten Armsegments des Haltearms;
- Figur 7: den Haltearm aus Figur 4 mit einer externen Steuereinheit und einem Manipulator gekoppelt;
- Figur 8: eine Draufsicht auf die Schnittstelle am proximalen Ende des Haltearms;
- Figur 9: eine perspektivische Ansicht eines externen Energiespeichers;
- Figur 10: eine perspektivische schematische Darstellung des ersten Armsegments mit einer mechanischen Schnittstelle zur Kopplung des Haltearms an eine Daumschiene eines Operationstisches;
- Figur 11: eine perspektivische Darstellung des siebten Armsegments samt Schnittstelle am distalen Ende;
- Figur 12: eine Draufsicht auf die Schnittstelle am distalen Ende des Haltearms;
- Figur 13: ein Flussdiagramm für ein Verfahren.

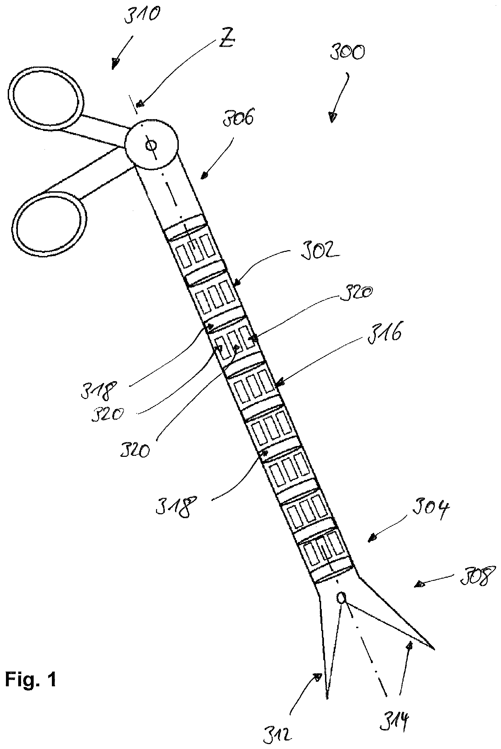

Gemäß Figur 1 weist ein chirurgisches Instrument 300 einen Schaft 302 zur Einführung in eine Körperöffnung eines Patienten auf. Der Schaft 302 weist einen distalen Abschnitt 304 sowie einen proximalen Abschnitt 306 auf. Der Schaft 302 ist insgesamt im Wesentlichen stabförmig, zylindrisch und starr ausgebildet. Die Erfindung ist allerdings nicht auf Starre und zylindrische Schäfte beschränkt, sondern auch bei flexiblen Schäften, die nicht zylindrisch geformt sind, anwendbar. An dem distalen Ende 304 ist eine Eingriffseinheit 308 angeordnet, die gemäß diesem Ausführungsbeispiel als Schere ausgebildet ist. Weiterhin weist das Instrument einen Handgriff 310 auf, der im Wesentlichen entsprechend eine üblichen Scherenhandgriff gebildet ist und mittels dem die beiden Scherenblätter 312, 314 gegeneinander verschwenkbar sind.

Auf einer äußeren Oberfläche weist der Schaft 302 eine Maßverkörperung 316 auf. Die Maßverkörperung 316 weist eine Mehrzahl an Breitengraden 318 (in Figur 1 nur zwei mit Bezugszeichen versehen) sowie eine Mehrzahl an Längengraden 320 (in Figur 1 nur drei mit Bezugszeichen versehen) auf. Die Längen- und Breitengrade 318, 320 sind gemäß diesem Ausführungsbeispiel aufmagnetisiert und durch einen Magnet-Sensorkopf erfassbar. In Figur 1 sind die Längen- und Breitengrade 318, 320 nur schematisch dargestellt, die Auflösung ist tatsächlich größer, nämlich 1mm. Möglich und bevorzugt ist auch eine Auflösung in einem Bereich von 0,1mm bis 0,8mm.

Die Breitengrade 318 dienen dazu, eine Eindringtiefe entlang der Zentralachse Z des Instruments zu erfassen, während die Längengrade 320 dazu dienen, eine Drehposition um die Achse Z herum zu erfassen.

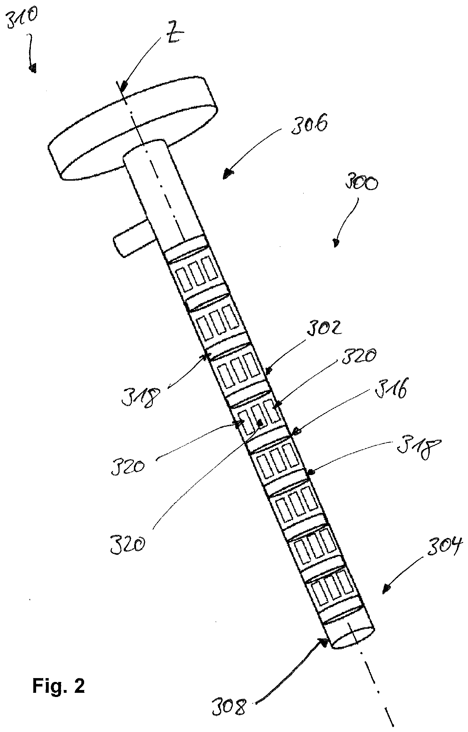

Figur 2 zeigt ein chirurgisches Instrument 300 gemäß einer zweiten Ausführungsform. Gleiche und ähnliche Elemente sind mit gleichen Bezugszeichen versehen und insofern wird vollumfänglich auf die obige Beschreibung bezüglich Figur 1 verwiesen. Das chirurgische Instrument gemäß Figur 2 ist als Endoskop ausgebildet und weist wiederum einen Schaft 302 zur Einführung in eine Körperöffnung eines Patienten auf. Der Schaft 302 hat ein distales Ende 304 und ein proximales Ende 306. An dem distalen Abschnitt 304 ist eine Eingriffseinheit 308 ausgebildet, die gemäß diesem Ausführungsbeispiel als Optik des Endoskops ausgebildet ist. Das Instrument 300 gemäß Figur 2 weist wiederum einen Handgriff 310 auf zur Bedienung des Endoskops. Der Schaft 302 ist im Wesentlichen zylindrisch ausgebildet und starr und hat eine Zentralachse

Auf dem Schaft 302 ist eine Maßverkörperung 316 angeordnet, die identisch zu der Maßverkörperung 316 aus Figur 1 ausgebildet ist. Sie weist eine Mehrzahl an Breitengraden 318 auf (in Figur 2 nur zwei mit Bezugszeichen versehen) sowie eine Mehrzahl an Längengraden 320 (in Figur 2 nur drei mit Bezugszeichen versehen). Die Maßverkörperung 316 ist wiederum aufmagnetisiert, wie mit Bezug auf Figur 1 bereits beschrieben wurde. Aufgrund der Maßverkörperung 316 ist die axiale Position als auch die Drehposition des Endoskops 300 relativ zu einer Sensoreinrichtung erfassbar.

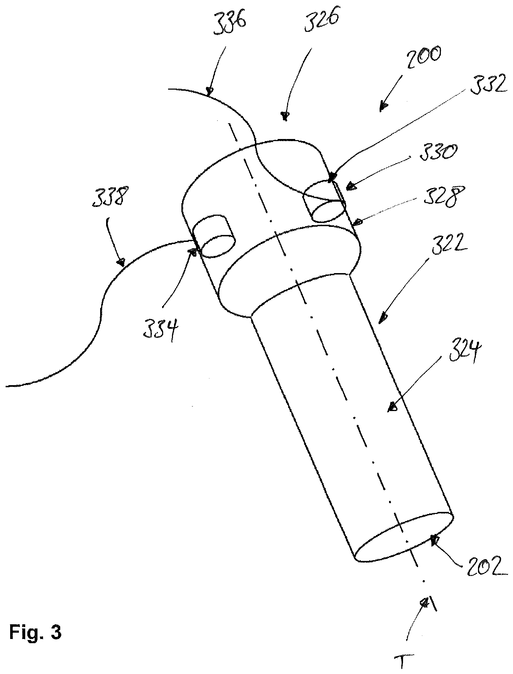

Figur 3 zeigt schematisch einen Manipulator 200. Dieser ist gemäß diesem Ausführungsbeispiel als Trokar ausgebildet und weist einen Grundkörper 322 auf. Der Grundkörper 322 hat einen im Wesentlichen zylindrischen hülsenförmigen Abschnitt 324, der ein offenes Arbeitsende 202 hat, aus welchem der Eingriffsabschnitt 308 des Instruments 300 herausragen kann, wenn dieses in den Trokar 200 eingesetzt ist. Dazu wird der Eingriffsabschnitt 308 von einer Eingangsöffnung 326 im Wesentlichen koaxial zur Zentralachse T des Trokars 200 eingeführt, sodass es an der Arbeitsöffnung 202 heraussteht.

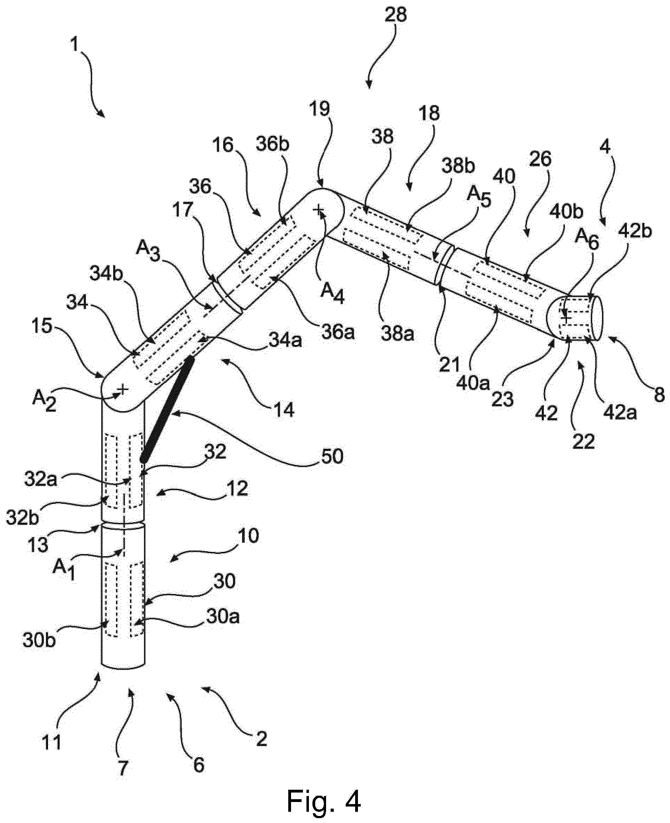

In einem Kopfabschnitt 328 des Trokars 200, mittels dem dieser auch an einem Haltearm befestigbar ist, ist eine Sensoreinrichtung 330 vorgesehen. Die Sensoreinrichtung weist einen ersten Magnetsensorkopf 332 und einen zweiten Magnetsensorkopf 334 auf. Beide Sensorköpfe 332, 334 sind mit Signalleitungen 336, 338 über einer Schnittstelle am distalen Ende des Haltearms und einem internen Bussystem 76 des Haltearms 1 gekoppelt (vgl. Figuren 4-6) und so mit einer Auswähleinheit und Steuereinheit 74 verbunden.

Die von den Sensorköpfen 332, 334 erfassten Daten können über das Bussystem 76 des Haltearms 1 auch an ein Navigationssystem des Operationssaals übermittelt werden. Indem zwei Sensorköpfe 232, 234 vorgesehen sind, kann die Auflösung der Sensoreinrichtung 330 mittels einer Umrechnung erhöht werden, indem die Sensorköpfe 332, 334 sowohl in axialer als auch in umfänglicher Richtung nicht in ganzzahligen Mehrfachen der Auflösung der Maßverkörperung 316 angeordnet sind, sondern beispielsweise um einen 0,9fachen Wert.

In anderen Ausführungsbeispielen ist die Maßverkörperung 316 (vgl. Figuren 1 und 2) optisch abtastbar. In solchen Fällen sind die Sensorköpfe 332, 334 als optische Erfassungsmittel, beispielsweise Laserscanner oder dergleichen ausgebildet.

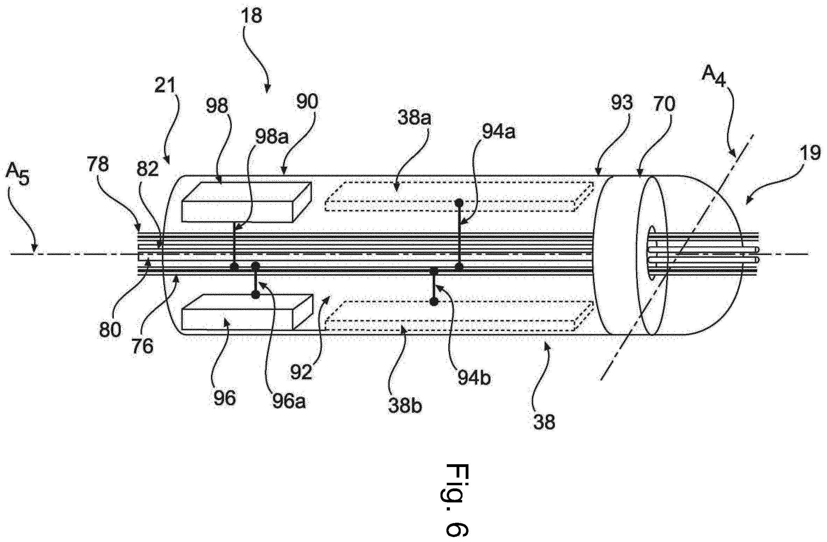

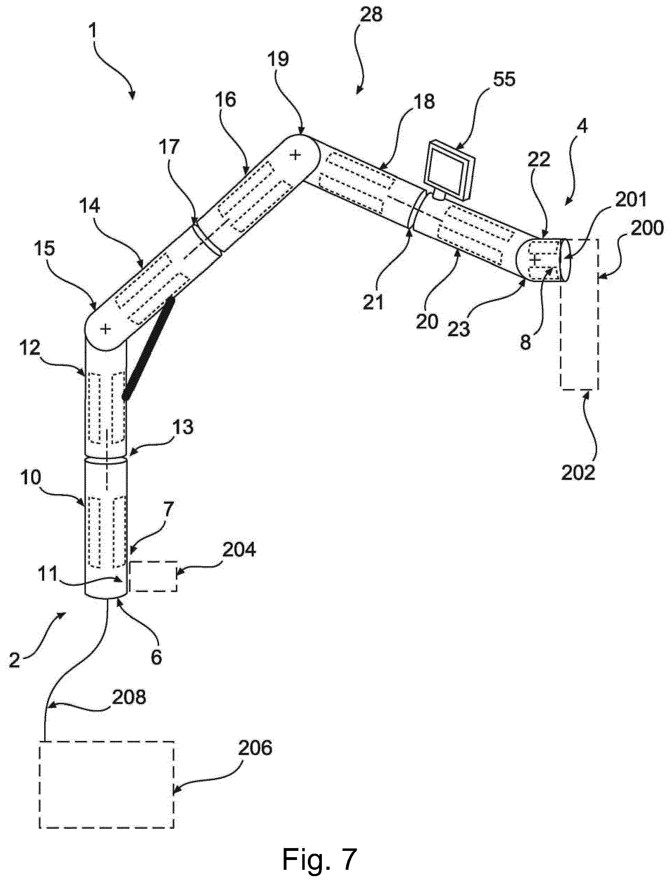

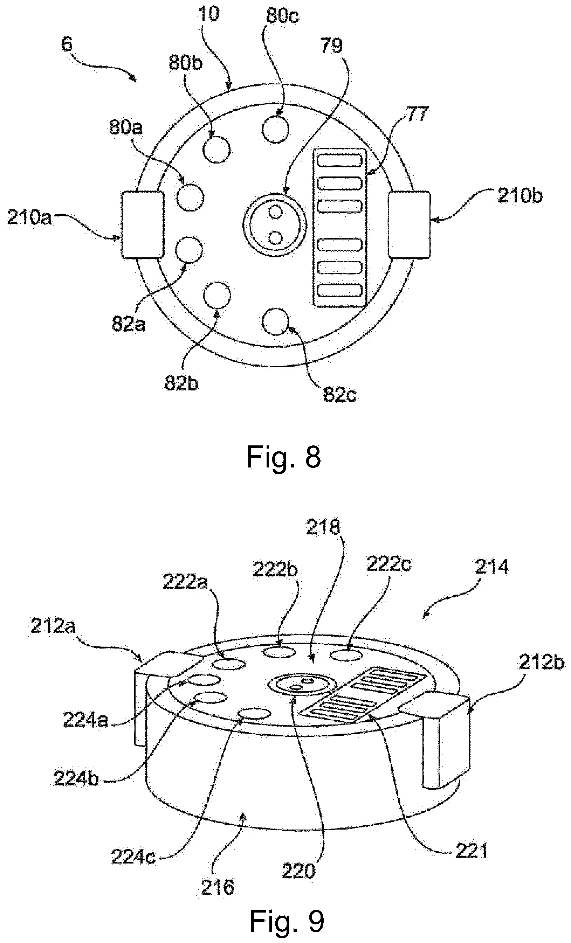

Figur 4 zeigt allgemein einen Haltearm 1 für medizinische Zwecke, insbesondere zum Halten eines chirurgischen mechatronischen Assistenzsystems und/oder chirurgischen Instruments, insbesondere Manipulator 200, wie in DE 10 2014 016 823 offenbart. Der Haltearm 1 weist ein proximales Ende 2 und ein distales Ende 4 auf. An dem proximalen Ende 2 sind eine erste Schnittstelle 6 und eine mechanische Schnittstelle 7 ausgebildet, welche mit Bezug auf die Figuren 6 und 8 genauer beschrieben werden. Die Schnittstelle 7 dient dazu, den Haltearm 1 an einer Basis, wie insbesondere an einem Operationstisch, zu befestigen. Die Schnittstelle 7 dient zur Übergabe von Energie sowie zur Kopplung des Haltearms 1 mit einer externen Steuereinheit (vgl. Fig. 7). An dem distalen Ende 4 ist eine zweite Schnittstelle 8 vorgesehen, über die ein mechatronisches Assistenzsystem und/oder ein chirurgisches Instrument, wie insbesondere ein Manipulator, an den Haltearm 1 koppelbar ist. Vorzugsweise wird hier ein Manipulator zum Halten und Manipulieren eines Endoskops angeordnet.

Der Haltearm 1 gemäß Figur 4 weist sieben Armsegmente 10, 12, 14, 16, 18, 20, 22 auf, die jeweils im Wesentlichen stabförmig sind und bis auf das letzte Armsegment 22 alle im Wesentlichen eine gleiche Länge aufweisen. Die sieben Armsegmente 10, 12, 14, 16, 18, 20, 22 sind jeweils mit Gelenken 11, 13, 15, 17, 19, 21, 23 miteinander gekoppelt, wobei das nullte Gelenk 11 den Haltearm 1 mit der Basis (in Fig. 4 nicht gezeigt, s. Fig. 9) koppelt. Die Gelenke 13, 15, 17, 19, 21, 23 sind gemäß diesem Ausführungsbeispiel sämtlich als rotatorische Gelenke mit jeweils einem Freiheitsgrad ausgebildet. Gemäß diesem Ausführungsbeispiel ist dem nullten Segment 10 das nullte Gelenk 11 zugeordnet, dem ersten Armsegment 12 das erste Gelenk 13 zugeordnet, dem zweiten Armsegment 14 das zweite Gelenk 15 zugeordnet, dem dritten Armsegment 16 das dritte Gelenk 17 zugeordnet, dem vierten Armgelenk 18 das vierte Gelenk 19 zugeordnet, dem fünften Armsegment 20 das fünfte Gelenk 21 zugeordnet, und dem sechsten Armsegment 22 ist das sechste Gelenk 23 zugeordnet. Das Gelenk 11 ist als translatorisches Gelenk ausgebildet, sodass das Armsegment 10 teleskopartig verlängerbar ist, um so den Haltearm 1 in der Höhe zu verstellen, wie später mit Bezug auf Figur 8 erläutert werden wird. Die Gelenke 13, 15, 17, 19, 21, 23 weisen jeweils Schwenkachsen A₁, A₂, A₃, A₄, A₅, A₆ auf, wobei jeweils benachbarte Gelenke Schwenkachsen haben, die senkrecht zueinander sind. Hierdurch wird eine einfache Positionierung des distalen Endes 4 im Raum erreicht.

Der Haltearm 1 gemäß Figur 4 weist ferner eine Bedieneinrichtung 28 auf. Mittels der Bedieneinrichtung 28 ist der Haltearm 1 in eine gewünschte Pose verbringbar, wobei die Bedieneinrichtung 28 dazu eingerichtet ist, bei Kontakt zwischen einer Bedienperson und einem der sieben Armsegmente das zugeordnete Gelenk freizugeben. Dazu weist die Bedieneinrichtung 28 gemäß diesem Ausführungsbeispiel sieben Kontaktabschnitte 30, 32, 34, 36, 38, 40, 42 auf, wobei an jedem Armsegment 10, 12, 14, 16, 18, 20, 22 jeweils ein Kontaktmittel 30, 32, 34, 36, 38, 40, 42 angeordnet ist. So ist am nullten Armsegment 10 ein nulltes Kontaktmittel 30 angeordnet, am ersten Armsegment 12 ein erstes Kontaktmittel 32, am zweiten Armsegment 14 ein zweites Kontaktmittel 34, am dritten Armsegment 16 ein drittes Kontaktmittel 36, am vierten Armsegment 18 ein viertes Kontaktmittel 38, am fünften Armsegment 20 ein fünftes Kontaktmittel 40 und am sechsten Armsegment 22 ein sechstes Kontaktmittel 42 angeordnet.

Ferner ist gemäß diesem Ausführungsbeispiel vorgesehen, dass jedes Kontaktmittel 30, 32, 34, 36, 38, 40, 42 jeweils zwei im Wesentlichen gegenüberliegend angeordnete Kontaktmittelelemente 30a, 30b, 32a, 32b, 34a, 34b, 36a, 36b, 38a, 38b, 40a, 40b, 42a, 42b aufweist. Die Kontaktmittel 30, 32, 34, 36, 38, 40, 42 dienen dazu, einen Kontakt eines Bedieners mit dem entsprechenden Armsegment 10, 12, 14, 16, 18, 20, 22 zu erfassen. Beim Ergreifen eines Armsegments 10, 12, 14, 16, 18, 20, 22 kommt der Bediener in Kontakt mit beiden Kontaktmittelelementen 30a, 30b bis 42a, 42b, und nur bei Kontakt mit beiden Kontaktmittelelementen 30a, 30b bis 42a, 42b eines Kontaktmittels 30 bis 42 wird das zugeordnete Gelenk freigegeben. Das heißt, beim Ergreifen des ersten Armsegments 12 und gleichzeitigem Inkontaktkommen mit den beiden Kontaktmittelelementen 32a, 32b wird durch die Bedieneinrichtung 28 das erste Gelenk 13 freigegeben. Dadurch ist es möglich, dass der Bediener den Haltearm 1 beziehungsweise die Armsegmente 12 bis 22 um die Achse A₁ verschwenken kann. Bei Loslassen eines der beiden oder beider Kontaktmittelelemente 32a, 32b wird das Gelenk 13 wieder arretiert, und ein Verschwenken um die Achse A₁ ist nicht mehr möglich. Auch bei einer unbeabsichtigten Berührung nur eines der beiden Kontaktmittelelemente 32a, 32b, beispielsweise mit einem Arm oder Ellenbogen des Bedieners, wird das Gelenk 13 nicht freigegeben, und der Haltearm 1 bleibt im arretierten Zustand und hält seine Pose.

Entsprechendes gilt für das zweite Armsegment 14. Auch hier weist das zweite Kontaktmittel 34 zwei Kontaktmittelelemente 34a, 34b auf, die im Wesentlichen gegenüberliegend umfänglich an dem Armsegment 14 vorgesehen sind. Bei Ergreifen dieses Armsegments 14 und Inkontaktkommen mit den beiden Kontaktmittelelementen 34a, 34b wird dieser Kontakt durch die Bedieneinrichtung 28 erfasst, und das dem Armsegment 14 zugeordnete Gelenk 15 freigegeben. Nun ist ein Verschwenken um die Achse A₂ möglich, sodass das distale Ende 4, bezogen auf Figur 4, nach oben beziehungsweise unten verschwenkt werden kann. Gleichzeitig bleiben alle weiteren Gelenke 13, 17, 19, 21, 23 arretiert, sodass in diesen keine Bewegung stattfindet.

Die Bedieneinrichtung 28 kann dazu einen Controller oder Mikroprozessor aufweisen, der dazu eingerichtet ist, einen Kontakt zwischen Kontaktmittelelementen 30a, 30b bis 42a, 42b zu erfassen und in elektrische Signale zu übertragen.

Die Kontaktmittel 30 beziehungsweise die Kontaktmittelemente 30a, 30b bis 42a, 42b sind gemäß diesem Ausführungsbeispiel als berührungsempfindliche Sensoren ausgebildet und erfassen einen Druck eines Kontakts zwischen dem Bediener und dem entsprechenden Kontaktmittelelement 30a, 30b bis 42a, 42b. Vorzugsweise sind die Kontaktmittelelemente 30a, 30b bis 42a, 42b als kapazitive berührungsempfindliche Sensoren ausgebildet.

Bei dem dargestellten Haltearm 1 ist es auch möglich, dass ein Bediener zwei Armsegmente, beispielsweise das Armsegment 14 und das Armsegment 18, gleichzeitig ergreift und so gleichzeitig die Kontaktmittelelemente 34a, 34b und 38a, 38b kontaktiert. Folglich werden die Gelenke 15 und 19 freigegeben, und ein Verschwenken sowohl um die Achse A₂ als auch um die Achse A₄ ist möglich. Bei dieser gleichzeitigen Freigabe ist es möglich, eine Winkelorientierung der Armsegmente 18 und 20 im Raum beizubehalten, während nur die Armsegmente 34, 36 verschwenkt werden. So ist auch eine translatorische Bewegung des distalen Endes 4 möglich. In einer bevorzugten Ausgestaltung des Haltearms werden bei dem gleichzeitigen Kontaktieren von zwei Armsegmenten, gemäß diesem Beispiel der Armsegmente 14 und 18 nicht die Gelenke 15 und 19 freigegeben, sondern alle zwischen diesen Armsegmenten 14 und 18 liegenden Gelenke, also gemäß diesem Ausführungsbeispiel die Gelenke 17 und 19. Das Gelenk 15 bleibt arretiert. Der Haltearm 1 kann nun so in seiner Pose verändert werden, dass eine Rotation um die Achse A3 und die Achse A4 möglich ist. Dies ist eine besonders intuitive Bedienung des Haltearms. Entsprechend werden, beispielsweise bei dem Kontakt zwischen Bediener und den Haltearmsegmenten 12 und 20, die Gelenke 15, 17, 19 und 21 freigegeben.

Weiterhin ist in der Figur 4 zu erkennen, dass der Haltearm 1 über eine Gewichtskompensationseinrichtung 50 verfügt. Die Gewichtskompensationseinrichtung 50 weist gemäß diesem Ausführungsbeispiel ein Gasdruckfederelement auf, welches mit dem Armsegment 14 und dem Armsegment 12 gekoppelt ist. Alternativ kann die Gewichtskompensationseinrichtung auch einen Seilzug und/oder ein ausbalanciertes Gegengewicht aufweisen. Bei dem Haltearm 1 gemäß Figur 4 lastet auf dem Gelenk 15 um seine Rotationsachse A2 das größte Moment. Folglich ist es bevorzugt, genau dieses Gelenk 15 mittels der Gewichtskompensationseinrichtung 50 abzustützen. Bei Freigabe des Gelenks 15, durch Kontaktieren des Armsegments 14, wird also ein Gewicht, welches auf dem Armsegment 14 lastet, aufgrund der weiteren Armsegmente 16, 18, 20, 22 und eines an der Schnittstelle 8 angeordneten Manipulators durch die Gewichtskompensationseinrichtung 50 abgestützt, sodass das distale Ende 4 bei Ergreifen des Segments 14 nicht sofort "absackt".

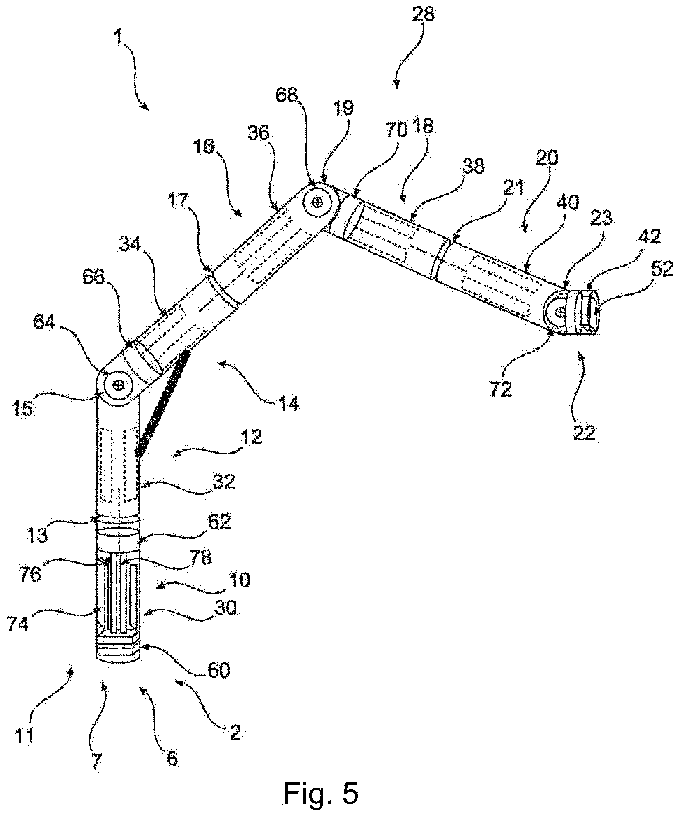

Der Haltearm 1 (vgl. Fig. 5) weist eine Erkennungseinheit 52 zum Erkennen eines mit der zweiten Schnittstelle 8 gekoppelten Assistenzsystems bzw. Manipulator 200 auf, wobei die Bedieneinrichtung 28 dazu angepasst ist, die Gelenke 11, 13, 15, 17, 19, 21, 23 in Abhängigkeit des mit der zweiten Schnittstelle 8 gekoppelten Manipulators 200 freizugeben oder zu arretieren. Eine derartige Erkennungseinheit 52 weist vorzugsweise einen Barcode-Scanner, einen QR-Code-Scanner oder einen RFID-Scanner auf. Vorzugsweise ist ein an der zweiten Schnittstelle 8 gekoppeltes Assistenzsystem mit einem entsprechenden Barcode, QR-Code oder einem RFID-Chip ausgestattet, welcher eine Identifikation des Manipulators 200 sowie vorzugsweise Informationen über dieses enthält. Hierdurch ist der Haltearm 1 in der Lage, zu erkennen, welcher Manipulator 200 an der zweiten Schnittstelle 8 gekoppelt ist und so ein Freigeben bestimmter Gelenke 11, 13, 15, 17, 19, 21, 23 teilweise oder vollständig zu verhindern. Beispielsweise ist an der zweiten Schnittstelle 8 mittels eines Manipulators 200 ein Endoskop 300 angeordnet. Der RFID-Chip des Endoskops 300 enthält Informationen über dieses Endoskop 300, wie insbesondere die geometrischen Abmaße des Endoskops 300. Diese Informationen werden von der Erkennungseinheit erkannt und an die Bedieneinrichtung 28 des Haltearms 1 und/oder die externe Steuereinheit weitergegeben. Die Bedieneinrichtung 28 ist dazu ausgebildet, solche Posen des Haltearms 1 zu verhindern, in welchen das Endoskop 300 mit dem Haltearm kollidieren würde. Ferner kann die Bedieneinrichtung 28 zusätzlich dazu ausgebildet sein, solche Posen des Haltearms 1 zu verhindern, in denen das Endoskop mit beispielsweise einem Operationstisch oder weiteren Gegenständen kollidieren würde. Durch die Sensoreinrichtung 330 und die Maßverkörperung 316 auf dem Endoskop 300 ist dann auch die axiale sowie die Drehposition des Endoskops 300 bezogen auf den Manipulator 200 bekannt.

In Figur 5 sind bei dem Haltearm 1 zusätzlich zu den bereits in Figur 4 gezeigten Elementen die Bremsen 60, 62, 64, 66, 68, 70, 72 dargestellt, mittels derer die Gelenke 11, 13, 15, 17, 19, 21, 23 freigebbar und arretierbar sind. Gleiche und ähnliche Elemente sind mit gleichen Bezugszeichen wie in Figur 4 versehen, und insofern wird vollumfänglich auf die obige Beschreibung Bezug genommen. Auch wenn in Figur 5 die Bezugszeichen an den Kontaktmittelelementen der Kontaktmittel 30, 32, 34, 36, 38, 40, 42 aus Klarheitsgründen nicht gezeigt sind, sind diese gleichwohl vorhanden, wie sich aus einem Vergleich der Figuren 4 und 5 ergibt.

Jedem Gelenk 11, 13, 15, 17, 19, 21, 23 ist jeweils eine Bremse 60, 62, 64, 66, 68, 70, 72 zugeordnet. Dem Gelenk 11 ist die Bremse 60 zugeordnet, dem Gelenk 13 die Bremse 62, dem Gelenk 15 die Bremse 64, dem Gelenk 17 die Bremse 66, dem Gelenk 19 die Bremse 68, dem Gelenk 21 die Bremse 70 und dem Gelenk 23 die Bremse 72. Alle Bremsen 60 bis 72 sind als elektromagnetische Bremsen mit einem Permanentmagnet ausgebildet, derart, dass diese unbestromt in einen arretierten Zustand vorgespannt sind. Der Permanentmagnet ist derart ausgelegt, dass dieser das jeweilige Gelenk alleine arretieren kann und die Pose des Haltearms 1 gehalten wird. In dem nullten Armsegment 10 ist eine elektronische Steuereinheit 74 vorgesehen. Diese ist über ein Bussystem 76 (in Figur 5 nur im Armsegment 10 gezeigt; vgl. Fig. 6) mit allen Kontaktmitteln 30 bis 42 der Bedieneinrichtung 28 sowie mit allen Bremsen 60 bis 72 gekoppelt. Zur Energieversorgung der Bremsen 60 bis 72 sowie der Kontaktmittel 30 bis 42 ist ferner eine Energieleitung 78 vorgesehen, die über die Schnittstelle 6 am proximalen Ende 2 des Haltearms 1 mit einer Energiequelle koppelbar ist.

Die Figur 6 zeigt eine Ausführungsform eines Armsegments, wobei in Figur 6 nur exemplarisch das vierte Armsegment 18 dargestellt ist. Es soll erkannt werden, dass auch die anderen Armsegmente 10, 12, 14, 16, 20, 22 ebenso ausgebildet sein können.

Das Armsegment 18 weist einen Armsegmentkörper 90 auf (in den Fig. 4 und 5 nicht gezeigt; es soll verstanden werden, dass jedes Armsegment 10 bis 22 einen Armsegmentkörper hat), der gemäß Figur 6 im Wesentlichen stabförmig beziehungsweise zylindrisch ausgebildet ist. Im Inneren weist der Armsegmentkörper 90 einen Hohlraum 92 auf, in dem verschiedene Elemente, wie etwa die Bremse 70, angeordnet sind. Schematisch dargestellt in Figur 6 sind die Gelenke 19, 21 sowie die beiden Schwenkachsen A₄, A₅ der Gelenke 19, 21, die jeweils mit dem Haltearmsegment 18 zusammenwirken. Dem Haltearmsegment 18 ist das Gelenk 19 zugeordnet (vgl. obige Beschreibung zu den Fig. 4 und 5). Der Armsegmentkörper 90 weist eine äußere Oberfläche 93 auf, die im Wesentlichen zylindrisch ausgebildet ist. Der Armsegmentkörper 90 ist beispielsweise aus einem Metall wie insbesondere Aluminium oder Titan einer Legierung auf Basis von Aluminium oder Titan, oder einem Faserverbundwerkstoff, wie etwas GFK oder CFK, gebildet und vorzugsweise in Leichtbauweise ausgelegt.

Das Armsegment 18 weist gemäß Figur 6 das Kontaktmittel 38 auf, welches Teil der Bedieneinrichtung 28 ist (vgl. Fig. 4 und 5). Das Kontaktmittel 38 weist zwei Kontaktmittelelemente 38a, 38b auf, die als berührungsempfindliche Sensoren ausgebildet sind und flächenbündig in der äußeren Oberfläche 93 des Armsegments 18 angeordnet sind. Die beiden Kontaktmittelelemente 38a, 38b sind im Wesentlichen gegenüberliegend bezogen auf die Achse A₅ angeordnet, sodass ein Bediener beim Ergreifen des Armsegments 18 in Kontakt mit beiden Kontaktmittelelementen 38a, 38b kommt, wie dies oben beschrieben ist.

Die beiden Kontaktmittelelemente 38a, 38b sind mittels Leitungen 94a, 94b mit dem Bussystem 76 gekoppelt. Über das Bussystem 76 sind die Kontaktmittelelemente 38a, 38b mit der elektronischen Steuereinheit 74 (vgl. Fig. 5) gekoppelt und über diese wiederum mit der Bremse 70, sodass die Bremse 70 durch die Bedieneinrichtung 28 freigegeben wird, wenn ein Bediener mit den Kontaktmittelelementen 38a, 38b in Kontakt kommt.

Neben dem Bussystem 76 sind innerhalb des Armsegmentkörpers 90 ein Energieübertragungssystem 78 sowie ein Kabelkanal 80 und ein Arbeitskanal 82 angeordnet. Mittels des Energieübertragungssystems 78 sind die Kontaktmittelelemente 38a, 38b sowie die Bremse 70 an eine Energieversorgung angeschlossen.

Alternativ oder zusätzlich ist in jedem Armsegment ein Elektronikmodul 96 angeordnet, welches über eine Leitung 96a mit dem Bussystem 76 gekoppelt ist. In einem solchen Fall wirken die Kontaktmittelelemente 38a, 38b, welche über die Leitung 94a, 94b mit dem Datenbussystem 76 verbunden sind, nur mit dem Elektronikmodul 96 zusammen, welches den durch die Kontaktmittelelemente 38a, 38b erfassten Kontakt in ein Stellsignal für die Bremse 70 umwandelt und dieses Stellsignal über das Bussystem 76 an die Bremse 70 sendet zum Freigeben des Gelenks 19. Ist in jedem Armsegment ein derartiges Elektronikmodul 96 angeordnet, ist ein im Wesentlichen modularer Aufbau des Haltearms 1 realisiert, und die einzelnen Armsegmente 10 bis 22 sind unabhängig von der elektronischen Steuereinheit 74, welche im proximalen Armsegment 10 angeordnet ist.

Der Kabelkanal 80 dient dazu, Kabel, die vom proximalen Ende 2 bis zum distalen Ende 4 verlaufen, um insbesondere die Schnittstelle 8 zu versorgen, führen zu können. Der Arbeitskanal 82 dient dazu, Schläuche oder Lichtleiter und dergleichen aufzunehmen, die je nach Art des Manipulator, der an der Schnittstelle 8 angeordnet ist, benötigt werden. Ist beispielsweise an der Schnittstelle 8 ein Endoskop angeordnet, wird vorzugsweise durch den Arbeitskanal 82 ein Lichtleiter geführt, der ein Bild, welches eine Endoskopkamera aufnimmt, übertragen kann. Der Arbeitskanal 82 dient demnach dazu, je nach Anwendungsgebiet entsprechende Übertragungsmittel aufzunehmen.

Weiterhin ist in dem Armsegment 18 ein Sensor 98 angeordnet. Vorzugsweise ist in jedem Armsegment 10 bis 22 ein Sensor angeordnet, und es soll verstanden werden, dass die Sensoren in den Armsegmenten 10, 12, 14, 16, 20 und 22 ebenso ausgebildet sein können wie der Sensor 98 im Armsegment 18. Der Sensor 98 ist vorzugsweise als Beschleunigungssensor ausgebildet. Indem in jedem Armsegment ein solcher Beschleunigungssensor vorgesehen ist, ist es möglich, zu jeder Zeit die Pose des Haltearms 1 zu bestimmen. Dazu ist der Sensor 98 über eine Leitung 98a mit dem Datenbussystem 76 gekoppelt, sodass die von dem Sensor 98 erfassten Daten an die elektronische Steuereinheit 74 übertragen werden, welche dann aus allen Sensordaten aus allen Armsegmenten die Pose des Haltearms 1 bestimmt. Ferner ist durch das Vorsehen eines derartigen Sensors 98 auch die absolute und relative Position eines an der Schnittstelle 8 angeordneten Endeffektors beziehungsweise Manipulators bestimmbar. Auch ist es möglich, wenn der Haltearm 1 an einem Operationstisch angeordnet ist, eine Bewegung dieses Operationstischs zu erkennen. Erfassen alle Sensoren in allen Armsegmenten eine Bewegung in dieselbe Richtung, ist dies ein Indikator dafür, dass der gesamte Haltearm 1 unter Beibehaltung seiner Pose bewegt wurde, beispielsweise dadurch, dass der Operationstisch oder eine Operationstischplatte relativ zu einer Säule der Operationstisches rotiert oder verschoben wurde. Auch eine solche Bewegung ist mittels der Sensoren 98 erfassbar. Ferner können äußere Impulse, wie etwa Stöße auf den Haltearm 1, erfasst werden.

Figur 7 illustriert erneut den Haltearm 1, welcher bereits mit Bezug auf die Figuren 4 und 5 beschrieben worden ist. In Figur 7 ist der Haltearm 1 in ein System eingebunden dargestellt. An dem distalen Ende 4 ist mittels der Schnittstelle 8 ein Manipulator 200 angeordnet, welcher mit der Schnittstelle 8 über eine Schnittstelle 201 gekoppelt ist. Sowohl der Manipulator 200 als auch die Schnittstelle 201 sind in Figur 7 nur schematisch dargestellt (vgl. insb. Fig. 3). Der Manipulator 200 weist einen Arbeitsabschnitt 202 auf, aus dem beispielsweise die Spitze des Endoskops 300 herausragen kann. An dem proximalen Ende 2 ist der Haltearm 1 gemäß Figur 7 über die mechanische Schnittstelle 7 mit einer Basis 204 gekoppelt. Die Basis 204 ist hier ebenfalls nur schematisch dargestellt. Sie kann beispielsweise als eine Normschiene eines Operationstisches ausgebildet sein.

Die erste Schnittstelle 6 ist gemäß diesem Ausführungsbeispiel mit einer externen Steuereinheit 206 gekoppelt. Dazu ist die Schnittstelle 6 mittels eines Kabels 208 mit der externen Steuereinheit 206 verbunden. Die externe Steuereinheit 206 ist gemäß diesem Ausführungsbeispiel als OP-System ausgebildet, welches beispielsweise einen herkömmlichen Computer aufweist sowie eine Eingabe-Ausgabe-Schnittstelle zur Bedienung des OP-Systems. Das OP-System weist vorzugsweise Software-Komponenten auf, die dazu ausgebildet sind, Daten, die von dem Haltearm 1 an der Schnittstellt 6 übergeben werden, zu speichern und zu verarbeiten.

Je nach Ausgestaltung der Schnittstelle 6 kann auch vorgesehen sein, dass diese drahtlos mit dem OP-System 206 kommuniziert, beispielsweise über Bluetooth®, Wi-Fi® oder Ähnliches.

Gemäß diesem Ausführungsbeispiel weist der Haltearm 1 ferner ein Anzeigemittel 55 auf, welches gemäß diesem Ausführungsbeispiel als LCD Display ausgebildet ist. Das Anzeigemittel 55 ist mit einer Steuereinheit verbunden und zeigt Repräsentationen von Daten an, die an der ersten Schnittstelle 6 oder zweiten Schnittstelle 8 übergeben werden. Beispielsweise werden auf dem Anzeigemittel ein Gewicht des Manipulators 200, welches an der Schnittstelle 8 gekoppelt ist, oder die erfasste axiale und Drehposition eines Instruments 300, angezeigt. Alternativ wird auf dem Anzeigemittel eine Repräsentation der Pose des Haltearms dargestellt, mit entsprechenden Belastungen an einzelnen Gelenken. Weitere Möglichkeiten sind hier denkbar. Es ist auch denkbar, dass hier Warnhinweise angezeigt werden.

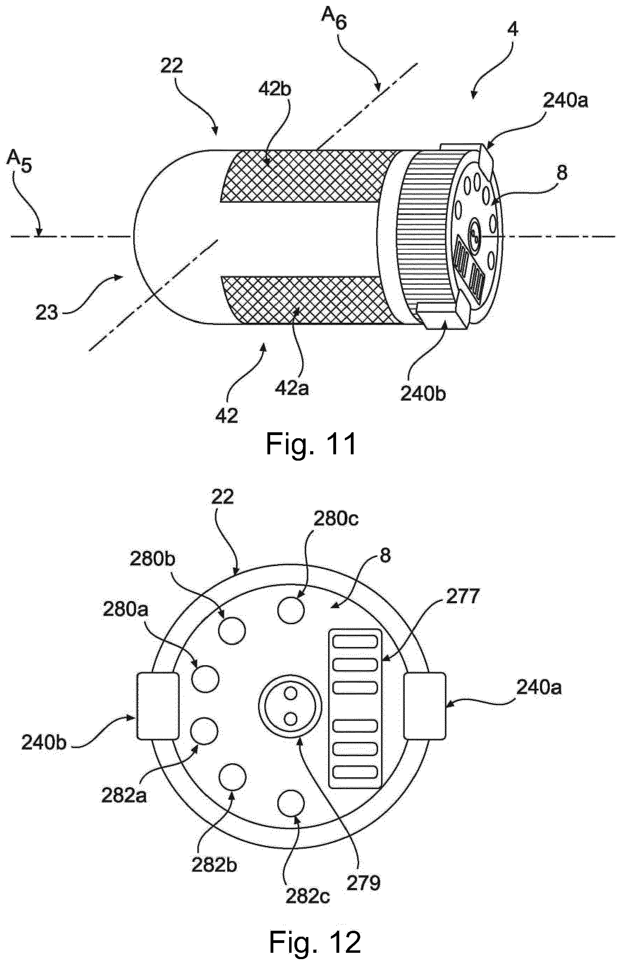

Die Schnittstelle 6 (s. Fig. 6) weist einen Anschluss 77 für das Bussystem 76 auf. Über diesen Anschluss 77 sind Daten, welche von Sensoren (vgl. Fig. 6) sowie den Kontaktmittelelementen (vgl. Fig. 5 - 6) an das Bussystem 76 gegeben werden, an die externe Steuereinheit 206 übertragbar. Der Anschluss 77 kann dazu als USB-Schnittstelle, RS-232-Schnittstelle, Bluetooth®-Schnittstelle, Wi-Fi®-Schnittstelle oder dergleichen ausgebildet sein. Mittig an der Schnittstelle 6 ist ferner ein Anschluss 79 zum Übertragen von elektrischer Energie vorgesehen. Mittels dieses Anschlusses 79 ist der Haltearm 1 mit einer Energiequelle, beispielsweise dem Stromnetz, koppelbar. Weiterhin sind an der Schnittstelle 6 drei Auslässe 80a, 80b, 80c des Kabelkanals 80 (vgl. obige Beschreibung zu den Fig. 5, 6) vorgesehen. Über diese Auslässe 80a, 80b, 80c sind Kabel, welche in den Kabelkanal 80 geführt sind, zugänglich. Zudem sind in der Schnittstelle 6 drei Auslässe 82a, 82b, 82c des Arbeitskanals 82 vorgesehen. Über die Auslässe 82a, 82b, 82c ist der Arbeitskanal 82 zugänglich. So ist beispielsweise mittels der Schnittstelle 6 ein Schlauch durch den Auslass 82b in den Arbeitskanal 82 führbar und durch diesen bis zur distalen Schnittstelle 8 (vgl. Fig. 12) führbar.

An einem umfänglichen Bereich des Armsegments 10 sind im Bereich der Schnittstelle 6 mechanische Kopplungsmittel 210a, 210b vorgesehen. Die Kopplungsmittel 210a, 210b korrespondieren mit Kopplungsmitteln 212a, 212b eines externen Energiespeichers 214 (s. Fig. 9). Der externe Energiespeicher 214 weist ein Gehäuse 216 auf, welches so gebildet ist, dass es sich proximal an das Armsegment 10 anfügen lässt. Der externe Energiespeicher 214 weist im Inneren Zellen zur Speicherung elektrischer Energie auf (in Fig. 9 nicht gezeigt). Der externe Energiespeicher 214 weist eine Schnittstelle 218 auf, die mit der Schnittstelle 6 des Haltearms 1 korrespondiert. Die Schnittstelle 218 weist einen Anschluss 220 auf, mittels dessen die in dem externen Energiespeicher 214 gespeicherte elektrische Energie über den Anschluss 79 der Schnittstelle 6 an den Haltearm 1 übertragbar ist. Ferner weist die Schnittstelle 218 einen Anschluss 221 auf, der mit der Schnittstelle 77 korrespondiert, zum Durchleiten von Signalen des Bussystems 76. Weiterhin weist der externe Energiespeicher Durchlässe 222a, b, c, 224a, b, c, auf, die mit den Auslässen 80a, b, c und 82a, b, c der Schnittstelle 6 korrespondieren, sodass durch den Kabelkanal 80 geführte Kabel auch durch den Energiespeicher 214 führbar sind sowie die Auslässe 82a, 82b, 82c des Arbeitskanals 80 ebenso an dem Energiespeicher 214 zugänglich sind.

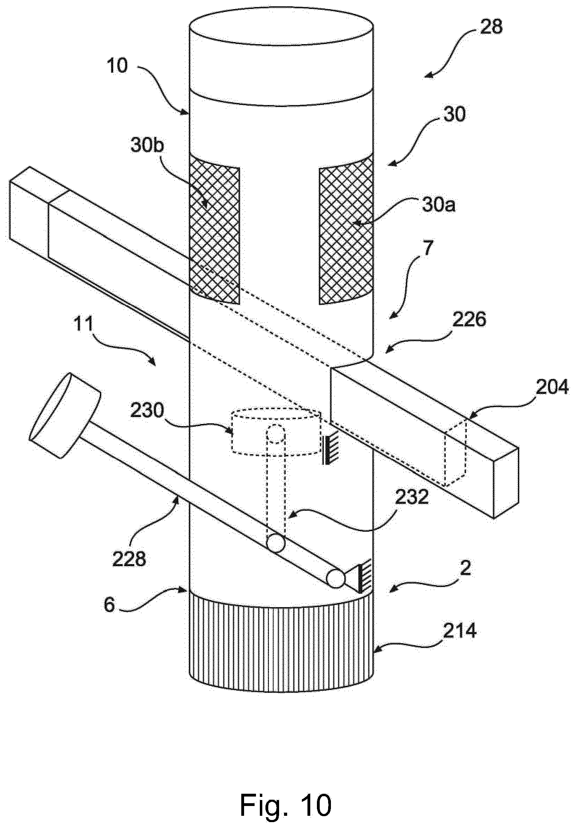

Fig. 10 illustriert das Armsegment 10, welches das proximale Ende 2 des Haltearms bildet, sowie insbesondere die mechanische Schnittstelle 7. An der ersten Schnittstelle 6 ist der externe Energiespeicher 214 (vgl. Fig. 9) angeordnet, sodass der Haltearm 1 gemäß diesem Ausführungsbeispiel (Fig. 10) autark betrieben werden kann, ohne die Notwendigkeit, diesen mit einer externen Energiequelle zu verbinden. Eine Verbindung zu einer Steuereinheit 206 kann dennoch vorgesehen sein und ist bevorzugt. Das Armsegment 10 weist ein Kontaktmittel 30, welches zwei Kontaktmittelabschnitte 30a, 30b hat, auf (vgl. auch Fig. 5). Die Schnittstelle 7 ist gemäß diesem Ausführungsbeispiel als eine Ausnehmung 226 gebildet, die an der äußeren Kontur der Basis 204, die hier als Normschiene eines Operationstisches ausgebildet ist, entspricht. Die Basis 204 ist in die Ausnehmung 226 führbar, und an dem Armsegment 10 sind mechanische Klemmmittel 228 vorgesehen, zum Klemmen des Armsegments 10 gegen die Basis 204. Die Klemmmittel weisen einen linear geführten Klemmkörper 230 auf, der über ein Gestänge 232 mit einem Hebel 224 antreibbar ist. Das Gelenk 11, welches über das Kontaktmittel 30 der Bedieneinrichtung 28 freigebbar ist, ist folglich als translatorisches Gelenk 11 ausgerichtet. Die Bedieneinrichtung ist dazu über ein elektrisches Stellmittel, welches in Figur 8 nicht gezeigt ist, mit dem Hebel 228 gekoppelt, sodass das Klemmmittel 230 außer Eingriff von der Basis 204 bringbar ist.

Die Figuren 11 und 12 schließlich illustrieren die zweite Schnittstelle 8 am distalen Ende 2 des Haltearms 1. Während Figur 11 die Schnittstelle 8 in einer perspektivischen Ansicht samt dem Armsegment 22 zeigt, zeigt Figur 10 die Schnittstelle 8 in einer Frontalansicht.

Die Schnittstelle 8 ist im Wesentlichen korrespondierend zur Schnittstelle 6 ausgebildet. An seitlichen Abschnitten von dieser, an dem Armsegment 22, sind zwei Sicherungselemente 240a, 240b angeordnet. Mittels der Sicherungselemente ist bestimmbar, ob ein an der Schnittstelle 8 gekoppeltes Assistenzsystem 200 (s. Fig. 7) korrekt mit der Schnittstelle 8 gekoppelt ist. Die Sicherungselemente 240a, 240b dienen gleichzeitig als mechanische Kopplungsmittel zum mechanischen Koppeln mit dem Manipulator 200, beispielsweise mittel Klemmung oder Verrastung.

An der Schnittstelle 8 ist ferner ein Anschluss 277 angeordnet, der mit dem Anschluss 77 der Schnittstelle 6 korrespondiert. Der Anschluss 277 ist mit dem Bussystem 76 gekoppelt, sodass Daten und Signale von dem Anschluss 77 über das Bussystem 76 zum Anschluss 77 übertragbar sind und umgekehrt. Ebenso ist ein Anschluss 279 an der Schnittstelle 8 vorgesehen, mittels dessen elektrische Energie von der Schnittstelle 8 auf den Manipulator 200 übertragbar ist. Der Anschluss 279 korrespondiert mit dem Anschluss 79 der Schnittstelle 6, und die beiden Anschlüsse 279 und 79 sind mittels des Übertragungsmittels 78 gekoppelt, zum Übertragen von elektrischer Energie zwischen diesen beiden Anschlüssen 79, 279.

Ferner sind an der Schnittstelle 8 Auslässe 280a, 280b, 280c des Kabelkanals 80 vorgesehen, sodass durch diesen geführte Kabel an der Schnittstelle 8 zugänglich sind. Das Gleiche gilt für den Arbeitskanal 82, von dem an der Schnittstelle 8 drei Auslässe 282a, 282b, 282c vorgesehen sind. Durch diese Schnittstelle 8 ist ein Manipulator 200 auf vorteilhafte Art und Weise mit dem Haltearm 1 koppelbar, ohne dass zusätzliche Übertragungsmittel oder Verkabelung an dem Haltearm 1 vorgesehen sein muss.

Wenn der Haltearm derart ausgebildet ist und an seinem distalen Ende beispielsweise ein Trokar mit einer Sensoreinrichtung 330 angeordnet ist ergeben sich überragende Vorteile. Dabei ist an dem Trokar vorzugsweise wenigstens ein Lokalisator für ein OP-Navigationssystem angeordnet. Von der Sensoreinrichtung 330 erfasste Daten können über den Haltearm in das OP-Navigationssystem eingespeist werden. Über das interne Bussystem können die Lageinformationen der Instrumente direkt an das OP-Navigationssystem übergeben werden. Dies wäre vor allem bei Eingriffen wichtig, bei denen häufige Instrumentenwechsel notwendig sind. Im Stand der Technik müssten in einem Solchen Fall alle Instrumente einen Lokalisator (optisch oder elektromagnetisch) aufweisen, der von der Navigationskamera des OP-Navigationssystems erkannt wird. Zusätzlich müssten unterschiedliche Instrumente unterscheidbare Lokalisatoren haben, damit die Navigationskamera diese unterscheiden kann. Im Falle der optischen Navigation unterscheiden sich die Lokalisatoren durch die Anordnung und Geometrie der optischen Reflektoren. Die Anzahl der unterschiedlichen Geometrien sind bei den bekannten optischen Navigationssystemen begrenzt (z.B. sechs Werkzeuge bei Polaris Vicra ®, der Fa. Northern Digital Inc., Waterloo, Canada). Gemäß der beschriebenen Ausführungsform sind an den Trokaren jeweils ein unterscheidbarer, fester oder abnehmbarer Lokalisator angeordnet. Dadurch ist jederzeit die Lage des Trokars in der OP-Navigation bekannt. Welches Instrument wie tief eingeführt wurde, und damit die Lage der Instrumentenspitze erfolgt dann über die die Sensoreinrichtung 330 in Verbindung mit der Maßverkörperung an dem Instrument. Die Trokare sind mit dem Haltearm bzw. den Haltearmen verbunden und bekommen die Daten und Informationen der Sensoreinrichtung 330 über das Bussystem des Haltearms mitgeteilt und stellen diese den angeschlossenen OP-Navigationssystemen anderer Hersteller an der proximalen Schnittstelle des Haltearms zur Verfügung. So können mit einer begrenzen Anzahl an chirurgischen Manipulatoren 20, wie etwa Trokaren, eine parktisch unbegrenzte Anzahl verschiedener Instrumente während einer Operation verwendet und im Navigationssystem berücksichtigt werden.

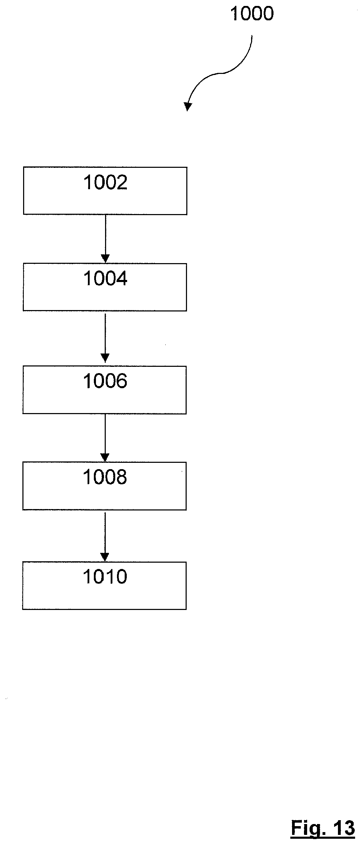

Figur 13 zeigt schematisch einen Ablauf eines Verfahrens 1000 zum Steuern eines chirurgischen Systems. Das Verfahren umfasst gemäß diesem Ausführungsbeispiel fünf Schritte. In einem ersten Schritt 1002 wird eine erste Position eines ersten Instruments 300 bezogen auf ein Referenzsystem bestimmt. Das Referenzsystem kann ein OP-Navigationssystem, der Patient oder ein anderes Referenzsystem, welches etwa mit dem OP-Tisch gekoppelt ist, sein. Anschließend oder gleichzeitig findet der Schritt Bestimmen 1004 einer ersten Position eines zweiten Instruments 300 bezogen auf dasselbe Referenzsystem statt. Es kann auch vorgesehen sein, dass zunächst auf ein anderes Referenzsystem bezogen bestimmt wird und anschließend eine Transformation in das erste Referenzsystem vorgenommen wird.

Anschließend, nachdem die ersten und zweiten Positionen in den Schritten 1002 und 1004 bestimmt wurden, folgt ein Schritt Erfassen 1006 einer gewünschten zweiten Position, in die das erste Instrument zu bewegen ist. Dies erfolgt vorzugsweise anhand eines Operationsplans und/oder eines Softwareprogramms, das einen Roboterarm, ein Instrument oder dergleichen steuert. Alternativ kann ein Operateur eine solche Position manuell eingeben oder das Instrument manuell in eine solche Position bewegen, wobei eine Auswerteeinheit und eine Steuereinheit vorzugsweise dazu eingerichtet sind, zu extrapolieren, in welche Richtung und zu welchem Punkt der Operateur das Instrument bewegt. Anschließend wird in Schritt 1008 eine erforderliche zweite Position und/oder Bewegung des zweiten Instruments bestimmt, in die das zweite Instrument zu verbringen oder zu bewegen ist, um einen Konflikt mit dem ersten Instrument zu verhindern, wenn dieses in die zweite Position bewegt wird. Im anschließenden Schritt 1010 wird das zweite Instrument in die in Schritt 1008 bestimmte Position bewegt. Dies kann wiederum manuell erfolgen, oder mittels eines Antriebs, der über die Auswerteeinheit und die Steuereinheit mit einem entsprechenden Stellsignal versorgt wird.

## Patentansprüche

1. Chirurgisches Instrument (300), insbesondere endoskopisches Instrument, mit einem Schaft (302) zur Einführung in eine Körperöffnung eines Patienten, der einen distalen und einen proximalen Abschnitt (304, 306) aufweist, wobei am distalen Abschnitt (304) eine Eingriffseinheit (308) angeordnet ist,
wobei der Schaft (302) eine Maßverkörperung (316) aufweist, die mittels einer Sensoreinrichtung zum Bestimmen einer Eindringtiefe und/oder Drehposition des Instruments abtastbar ist,
wobei die Maßverkörperung (316) eine Mehrzahl an um eine Zentralachse (Z) des Schafts (302) angeordnete Breitengrade (318) und eine Mehrzahl an parallel zu einer Zentralachse (Z) des Schafts (302) angeordnete Längengraden (320) aufweist, wobei eine Oberfläche des Schafts (302) ringförmig magnetisierte Abschnitte und magnetisierte Längsstreifen aufweist, wobei die ringförmig magnetisierten Abschnitte die Breitengrade bilden, wobei die magnetisierten Längsstreifen die Längengrade bilden, und wobei die Maßverkörperung (316) elektromagnetisch abtastbar ist.

2. Chirurgisches Instrument nach Anspruch 1 , wobei die Maßverkörperung (316) an einem Adapter ausgebildet ist, der auf dem Schaft (302) befestigt ist.

3. Chirurgischer Manipulator (200) zum Halten und Führen eines Instruments (300) nach einem der vorstehenden Ansprüche 1 oder 2, der vorzugsweise an einem Haltearm (1) befestigbar und mit diesem positionierbar ist,
wobei eine Sensoreinrichtung (330) vorgesehen ist, mittels der die Maßverkörperung (316) auf dem Schaft (302) des Instruments (300) zum Bestimmen einer Eindringtiefe und/oder Drehposition des Instruments (300) abtastbar ist, wobei die Sensoreinrichtung (330) wenigstens einen Magnet-Sensorkopf (332, 334) zum elektromagnetischen Erfassen der Maßverkörperung (316) aufweist,
wobei die Sensoreinrichtung (330) mit einer Steuereinheit (74) verbunden ist, die dazu eingerichtet ist, eine Funktion des Manipulators (200) und/oder des Instruments (300) in Abhängigkeit von durch die Sensoreinrichtung (330) erfassten Werten zu sperren oder freizugeben, um eine Fehlbedienung des Instruments zu verhindern.

4. Manipulator nach Anspruch 3, wobei die Sensoreinrichtung (330) wenigstens eine Kamera zum optischen Erfassen der Maßverkörperung aufweist.

5. Manipulator nach einem der Ansprüche 3 oder 4, aufweisend einen Trokar zur Aufnahme des Schafts (302) des Instruments (300), wobei die Sensoreinrichtung (330) an dem Trokar, insbesondere eine proximale Öffnung (326) des Trokars, angeordnet ist.

6. Manipulator nach einem der Ansprüche 3 bis 5, wobei die Sensoreinrichtung (330) nahe an einer Körperöffnung, insbesondere natürlichen Körperöffnung des Patienten positionierbar ist.

7. Manipulator nach einem der Ansprüche 3 bis 6, aufweisend eine Antriebs- und/oder Bremseinrichtung zum Antreiben und/oder Bremsen einer Bewegung des Instruments relativzum Manipulator (200).

8. Manipulator nach einem der Ansprüche 3 bis 7, ferner aufweisend einen Haltearm (1) zum Halten eines Instruments nach einem der Ansprüche 1 oder 2, wobei der Haltearm ein proximales Ende (2) zum Befestigen des Haltearms (1) an einer Basis und ein distales Ende (4) aufweist, in dem der Manipulator (200) aufgenommen ist;
wobei der Haltearm (1) wenigstens ein erstes und ein zweites Armsegment (12, 14) aufweist, wobei das erste Armsegment (12) mit einem ersten Gelenk (13) und das zweite Armsegment (14) mit einem zweiten Gelenk (15) verbunden ist, wobei jedes Gelenk (13, 15) freigebbar und arretierbar ist.

9. Chirurgisches System mit wenigstens zwei Manipulatoren (200) nach Anspruch 3 und zwei Instrumenten (300) nach Anspruch 1, wobei jeweils ein Instrument (300) in einem Manipulator (200) aufgenommen ist, und wobei die Sensoreinrichtungen (330) der Manipulatoren (200) mit einer Steuereinheit (74) verbunden sind, wobei die Steuereinheit (74) dazu ausgebildet ist eine Funktion des ersten Manipulators (200) und/oder des ersten Instruments (300) in Abhängigkeit von durch die ersten und zweiten Sensoreinrichtungen (330) erfassten Werten des ersten und zweiten Instruments (300) zu sperren oder freizugeben, um eine Fehlbedienung des ersten und/oder zweiten Instruments zu verhindern.

## Claims

1. A surgical instrument (300), in particular an endoscopic instrument, having a shaft (302) for insertion into a body orifice of a patient, the shaft comprising a distal and a proximal portion (304, 306), wherein an engagement unit (308) is arranged at the distal portion (304),
wherein the shaft (302) comprises a standard (316) scannable by means of a sensor device for determining a penetration depth and/or rotational position of the instrument,
wherein the standard (316) comprises a plurality of latitudes (318) arranged about a central axis (Z) of the shaft (302) and a plurality of longitudes (320) arranged parallel to a central axis (Z) of the shaft (302),
wherein a surface of said shaft (302) comprises annularly magnetized portions and magnetized longitudinal strips, wherein the annularly magnetized portions form the latitudes, wherein the magnetized longitudinal strips form the longitudes, and wherein the standard (316) is electromagnetically scannable.

2. The surgical instrument according to claim 1, wherein the standard (316) is formed on an adapter which is attached to the shaft (302).

3. A surgical manipulator (200) for holding and guiding an instrument (300) according to one of the preceding claims 1 or 2, which is preferably attachable to and positionable with a holding arm (1),
wherein a sensor device (330) is provided by means of which the standard (316) on the shaft (302) of the instrument (300) can be scanned for determining a penetration depth and/or rotational position of the instrument (300), wherein the sensor device (330) comprises at least one magnetic sensor head (332, 334) for electromagnetic detection of the standard (316),
wherein the sensor device (330) is connected to a control unit (74) which is adapted to disable or enable a function of the manipulator (200) and/orthe instrument (300) depending on values detected by the sensor device (330) to prevent incorrect operation of the instrument.

4. Manipulator according to claim 3, wherein the sensor device (330) comprises at least one camera for optically detecting the standard.

5. Manipulator according to one of claims 3 or 4, comprising a trocar for receiving the shaft (302) of the instrument (300), wherein the sensor device (330) is arranged on the trocar, in particular on a proximal opening (326) of the trocar.

6. Manipulator according to one of claims 3 to 5, wherein the sensor device (330) is positionable close to a body orifice, in particular to a natural body orifice of the patient.

7. Manipulator according to any one of claims 3 to 6, comprising driving and/or braking means for driving and/or braking a movement of the instrument relative to the manipulator (200).

8. Manipulator according to any one of claims 3 to 7, further comprising a holding arm (1) for holding an instrument according to any one of claims 1 or 2, the holding arm comprising a proximal end (2) for attaching the holding arm (1) to a base and a distal end (4) in which the manipulator (200) is received;
the holding arm (1) comprising at least a first and a second arm segment (12, 14), the first arm segment (12) being connected to a first joint (13) and the second arm segment (14) being connected to a second joint (15), wherein each joint (13, 15) is releasable and lockable.

9. Surgical system with at least two manipulators (200) according to claim 3 and two instruments (300) according to claim 1, wherein one instrument (300) is accommodated in each manipulator (200), and wherein the sensor devices (330) of the manipulators (200) are connected to a control unit (74), wherein the control unit (74) is adapted to disable or enable a function of the first manipulator (200) and/or the first instrument (300) depending on values of the first and second instruments (300) detected by the first and second sensor means (330) to prevent incorrect operation of the first and/or second instrument.

## Revendications

1. Instrument (300) chirurgical, notamment instrument endoscopique, comprenant un fût (302) à introduire dans une ouverture corporelle d'un patient, qui a une partie (304) distale et une partie (306) proximale, dans lequel une unité (308) de pénétration est disposée à la partie (304) distale,
dans lequel le fût a une mesure (316) matérialisée, qui peut être détectée au moyen d'un dispositif de capteur pour la détermination d'une profondeur de pénétration et/ou d'une position en rotation de l'instrument,
dans lequel la mesure (316) matérialisée a une pluralité de degrés (318) de largeur, disposés autour d'un axe (Z) central du fût (302), et une pluralité de degrés (320) de longueur, disposés parallèlement à un axe (Z) central du fût (302), une surface du fût (302) ayant des parties aimantées annulairement et des bandes longitudinales aimantées, les parties aimantées annulairement formant les degrés de largeur, les bandes longitudinales aimantées formant les degrés de longueur et la mesure (316) matérialisée pouvant être détectée électromagnétiquement.

2. Instrument chirurgical suivant la revendication 1, dans lequel la mesure (316) matérialisée est constituée sur un adaptateur, qui est fixé au fût (302).

3. Manipulateur (200) chirurgical pour maintenir et guider un instrument (300) suivant l'une des revendications 1 ou 2 précédentes, qui peut être fixé, de préférence, à un bras (1) de maintien et peut être mis en position par celui-ci,
dans lequel il est prévu un dispositif (330) de capteur, au moyen duquel la mesure (316) matérialisée sur le fût (302) de l'instrument (300) peut être détectée pour la détermination d'une profondeur de pénétration et/ou d'une position en rotation de l'instrument (300), le dispositif (330) de capteur ayant au moins une tête (332, 334) de capteur à aimant pour la détection électromagnétique de la mesure (316) matérialisée,
dans lequel le dispositif (330) de capteur est relié à une unité (74) de commande, qui est conçue pour bloquer ou valider un fonctionnement du manipulateur (200) et/ou de l'instrument (300) en fonction de valeurs détectées par le dispositif (330) de capteur, afin d'empêcher une commande défectueuse de l'instrument.

4. Manipulateur suivant la revendication 3, dans lequel le dispositif (330) de capteur a au moins un appareil photographique pour la détection optique de la mesure matérialisée.

5. Manipulateur suivant l'une des revendications 3 ou 4, comportant un trocart de réception du fût (302) de l'instrument (300), le dispositif (330) de capteur étant monté sur le trocart, notamment à une ouverture (326) proximale du trocart.

6. Manipulateur suivant l'une des revendications 3 à 5, dans lequel le dispositif (330) de capteur peut être mis en position à proximité d'une ouverture du corps, notamment d'une ouverture naturelle du corps du patient.

7. Manipulateur suivant l'une des revendications 3 à 6, comportant un dispositif d'entraînement et/ou de freinage pour l'entraînement et/ou le freinage d'un déplacement de l'instrument par rapport au manipulateur (200).

8. Manipulateur suivant l'une des revendications 3 à 7, comportant, en outre, un bras (1) de maintien pour maintenir un instrument suivant l'une des revendications 1 ou 2, le bras (1) de maintien ayant une extrémité (2) proximale pour la fixation du bras (1) de maintien à un socle et une extrémité (4) distale, dans lequel le manipulateur (200) est reçu; dans lequel le bras (1) de maintien a au moins un premier et un deuxième segments (12, 14) de bras, le premier segment (12) de bras étant relié à une première articulation (13) et le deuxième segment (14) de bras à une deuxième articulation (15), chaque articulation (13, 15) pouvant être libérée et bloquée.

9. Système chirurgical ayant au moins deux manipulateurs (200) suivant la revendication 3 et deux instruments (300) suivant la revendication 1, dans lequel, respectivement, un instrument (300) est reçu dans un manipulateur (200) et dans lequel les dispositifs (330) de capteur des manipulateurs (200) sont reliés à une unité (74) de commande, l'unité (74) de commande étant constituée pour bloquer ou valider un fonctionnement du premier manipulateur (200) et/ou du premier instrument (300), en fonction de valeurs, détectées par le premier et le deuxième dispositifs (330) de capteur, du premier et du deuxième instruments (300), afin d'empêcher une commande défectueuse du premier et/ou du deuxième instruments.
